(19) Europäisches Patentamt
European Patent Office
Office européen des brevets

(11) EP 4 785 926 A1

(12) EUROPEAN PATENT APPLICATION
published in accordance with Art. 153(4) EPC

(43) Date of publication:
**05.08.2026 Bulletin 2026/32**

(21) Application number: **24872251.4**

(22) Date of filing: **25.09.2024**

(51) International Patent Classification (IPC):
***A61K 8/894*** (2006.01) ***A61K 8/04*** (2006.01)
***A61K 8/19*** (2006.01) ***A61K 8/29*** (2006.01)
***A61K 8/34*** (2006.01) ***A61K 8/891*** (2006.01)
***A61Q 17/04*** (2006.01) ***A61Q 19/00*** (2006.01)

(52) Cooperative Patent Classification (CPC):
**A61K 8/04; A61K 8/19; A61K 8/25; A61K 8/26; A61K 8/29; A61K 8/34; A61K 8/58; A61K 8/891; A61K 8/893; A61K 8/894; A61Q 1/02; A61Q 17/04; A61Q 19/00**

(86) International application number:
**PCT/JP2024/034121**

(87) International publication number:
**WO 2025/070465 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 JP 2023167672**

(71) Applicant: **SHIN-ETSU CHEMICAL CO., LTD.**
**Tokyo 100-0005 (JP)**

(72) Inventors:
- **IMAI Taro**
  **Annaka-shi, Gunma 379-0224 (JP)**
- **MORIYA Hiroyuki**
  **Annaka-shi, Gunma 379-0224 (JP)**
- **KONISHI Masayuki**
  **Tokyo 100-0005 (JP)**
- **MIYAUCHI Masaru**
  **Tokyo 100-0005 (JP)**

(74) Representative: **Mewburn Ellis LLP**
**Aurora Building**
**Counterslip**
**Bristol BS1 6BX (GB)**

# (54) AQUEOUS DISPERSION AND COSMETIC PREPARATION CONTAINING SAME

(57) Disclosed is a cosmetic preparation which contains:
(a) 10% by mass to 70% by mass of hydrophobized titanium dioxide fine particles which have a number average primary particle diameter of 8 nm to 200 nm as determined by an image analysis method of a transmission electron micrograph, and are obtained by hydrophobizing titanium dioxide particles with silicone;
(b) 1.0% by mass to 30% by mass of an aqueous component which has two or more alcoholic hydroxyl groups;
(c) 1.0% by mass to 20% by mass of a polyglycerol-modified silicone which can be dissolved in the component (b); and
(d) 8% by mass to 82% by mass of water.
The cosmetic preparation has high stability and is excellent in terms of transparency, feeling of use (non-stickiness), and water resistance.

EP 4 785 926 A1

## Description

### TECHNICAL FIELD

The present invention relates to an aqueous dispersion containing hydrophobized fine particle of titanium dioxide, and to a cosmetic composition having the aqueous dispersion blended therein.

### BACKGROUND ART

In sunscreen cosmetic compositions, inorganic powders, such as titanium dioxide and zinc oxide, are usually used in the form of fine particle of inorganic powder for the purposes of improving transparency and enhancing UV-shielding effects (Patent Document 1). These inorganic powders, however, have the propensity that the particles strongly interact with one another due to the large surface area and consequently are aggregated easily.

These fine particle of inorganic powders are often hydrophobized on the surface so that the powders can be blended into an oil phase of a cosmetic composition or their water resistance will be enhanced. The addition of such surface-hydrophobized powders is expected to provide UV-shielding effects that can be maintained even when wetted with water (Patent Documents 2 and 3). On the other hand, a fine particle of inorganic powder is sometimes hydrophilized on the surface with silica or the like when the powder is to be blended into an aqueous phase. Unfortunately, such hydrophilized powders have drawbacks in water resistance and the feeling on use. Studies on dispersing hydrophobized powders in water have led to the use of a polyether-modified silicone in an aqueous dispersion of hydrophobized fine particle of titanium dioxide. However, such an aqueous dispersion of titanium dioxide has drawbacks in water resistance and the feeling on use (Patent Document 4).

Furthermore, a technique is known in which a dispersible powder is obtained by surface-treating a powder with a silicone surfactant. However, dispersibility and water resistance have not been studied sufficiently for the case where the powder is a hydrophobized inorganic powder (Patent Document 5).

In other studies, nonionic surfactants are added to dispersions, but this approach is unsuccessful in achieving sufficient dispersibility (Patent Document 6). For these reasons, there has been a demand for a dispersion that has good long-term stability and offers a good feeling on use when blended into a cosmetic composition.

### PRIOR ART DOCUMENTS

### PATENT DOCUMENTS

| | |
|---|---|
| Patent Document 1: | JP-A 2006-1886 |
| Patent Document 2: | JP-A 2014-201569 |
| Patent Document 3: | WO 2016/178380 |
| Patent Document 4: | WO 2015/125622 |
| Patent Document 5: | JP-A 2020-002031 |
| Patent Document 6: | JP-A H07-247119 |

### SUMMARY OF INVENTION

### TECHNICAL PROBLEM

The present invention has been made in consideration of the circumstances discussed above. It is therefore an object of the present invention to provide an aqueous dispersion in which a fine particle of inorganic powder has excellent dispersibility in an aqueous medium and exhibits high dispersion stability. Another object of the present invention is to provide a cosmetic composition that includes the aqueous dispersion and has high stability and excellent transparency, feeling on use (non-stickiness), and water resistance.

### SOLUTION TO PROBLEM

The present inventors carried out extensive studies directed to achieving the above objects. As a result, the present inventors have found that even titanium dioxide fine particles that are difficult to disperse can be dispersed in an

aqueous medium with excellent dispersibility and the aqueous dispersion exhibits high stability (viscosity stability), has long-term stability, and excels in the feeling on use (non-stickiness) and water resistance. The present inventors have found that when this dispersion is used as a dispersion in a cosmetic composition, the dispersion is easy to admit, and the cosmetic composition that is obtained has excellent transparency, feeling on use, and water resistance, thus completing the present invention.

Accordingly, the present invention provides an aqueous dispersion and a cosmetic composition as defined below.

1. An aqueous dispersion including:

    (a) 10 to 70% by weight of hydrophobized titanium dioxide fine particles including titanium dioxide particles hydrophobized with a silicone, the hydrophobized titanium dioxide fine particles having a number average primary particle diameter of 8 to 200 nm as determined by image analysis of a transmission electron photomicrograph;
    (b) 1.0 to 30% by weight of an aqueous component having at least two alcoholic hydroxyl groups;
    (c) 1.0 to 20% by weight of a polyglycerin-modified silicone that dissolves in the component (b); and
    (d) 8 to 82% by weight of water.

2. The aqueous dispersion according to 1, wherein the component (a) is a hydrophobized fine particle of titanium dioxide obtained by hydrophobizing hydrous silica-coated titanium dioxide particles.
3. The aqueous dispersion according to 1 or 2, wherein the silicone in the component (a) is hydrogen dimethicone.
4. The aqueous dispersion according to any one of 1 to 3, wherein the component (b) is an aqueous component having two alcoholic hydroxyl groups.
5. The aqueous dispersion according to any one of 1 to 4, wherein the component (c) is a component that does not dissolve in the component (d).
6. The aqueous dispersion according to any one of 1 to 5, wherein the component (c) is polyglyceryl-3 disiloxane dimethicone.
7. The aqueous dispersion according to any one of 1 to 6, wherein the weight ratio (c)/(b) of the content of the component (c) to the content of the component (b) is 0.2 to 0.9.
8. The aqueous dispersion according to any one of 1 to 7, wherein the total content of the components (a), (b), (c), and (d) is 90% by weight or more of the whole of the aqueous dispersion.
9. An aqueous dispersion including:

    (a) 10 to 70% by weight of hydrophobized titanium dioxide fine particles having a number average primary particle diameter of 8 to 200 nm as determined by image analysis of a transmission electron photomicrograph, the hydrophobized titanium dioxide fine particles being such that titanium dioxide particles selected from hydrous silica-coated titanium dioxide particles, and hydrous silica-coated and aluminum hydroxide-coated titanium dioxide particles are hydrophobized with a silicone selected from triethoxycaprylylsilane, dimethyl silicone, hydrogen dimethicone, triethoxysilylethyl polydimethylsiloxyethyl dimethicone, and triethoxysilylethyl polydimethylsiloxyethylhexyl dimethicone;
    (b) 1.0 to 30% by weight of at least one selected from BG (labeling name), DPG (labeling name), and glycerin;
    (c) 1.0 to 20% by weight of polyglyceryl-3 disiloxane dimethicone; and
    (d) 8 to 82% by weight of water.

10. A cosmetic composition including the aqueous dispersion described in any one of 1 to 9.

## ADVANTAGEOUS EFFECTS OF INVENTION

According to the present invention, an aqueous dispersion is obtained that has excellent dispersion properties in an aqueous medium, high stability (viscosity stability), long-term stability, excellent feeling on use (non-stickiness), and excellent water resistance. When used as a dispersion in a cosmetic composition, the aqueous dispersion is easy to admit, and the cosmetic composition that is obtained has excellent transparency, feeling on use, and water resistance.

## DESCRIPTION OF EMBODIMENTS

Now the present invention is described in detail. Herein, the name of ingredients is sometimes expressed by the Japanese labeling name or the International Nomenclature of Cosmetic Ingredients (INCI). When the Japanese labeling name is identical with the INCI, sometimes either one is omitted.

[Components (a)]

The component (a) in the present invention is hydrophobized titanium dioxide fine particles that include titanium dioxide particles hydrophobized with a silicone and have a number average primary particle diameter of 8 to 200 nm as determined by image analysis of a transmission electron photomicrograph. Such components (a) may be used singly, or two or more may be used in combination. Among titanium dioxide fine particles having UV-shielding effects and used as UV scattering agents, any titanium dioxide that can be usually added as an ingredient to cosmetic compositions may be used. The powder may be a composite powder of two or more of titanium dioxide, and zinc oxide and cerium oxide, or may be a composite powder of titanium dioxide with other powder.

In order to weaken aggregation or to reduce the activity of the powder, the titanium dioxide fine particles may be surface-treated with, for example, silica, hydrous silica, alumina, or aluminum hydroxide, before the hydrophobic treatment. The use of alumina or aluminum hydroxide as a surface treatment agent is not preferable in the case where there is a concern that such a surface treatment agent may inhibit the swelling of a water-soluble polymer compound added together with the powder in a cosmetic composition, or may cause a loss of water resistance. Hydrous silica-coated titanium dioxide particles that are surface-treated with hydrous silica are preferable because hydrous silica suppresses the activity of the powder and is unlikely to inhibit the swelling of a water-soluble polymer. The term "coated" means that the titanium dioxide particles are coated partially or completely.

In the present invention, the titanium dioxide particles are hydrophobized with a silicone. Examples of the silicones used for the hydrophobic treatment include silanes or silylating agents, such as triethoxycaprylylsilane (AES-3083, manufactured by Shin-Etsu Chemical Co., Ltd.), dimethyl silicone (KF-96AK series, manufactured by Shin-Etsu Chemical Co., Ltd.), methyl hydrogen polysiloxanes, such as hydrogen dimethicone (for example, KF-99P and KF-9901, manufactured by Shin-Etsu Chemical Co., Ltd.), and silicone-branched silicones, such as triethoxysilylethyl polydimethylsiloxyethylhexyl dimethicone and triethoxysilylethyl polydimethylsiloxyethylhexyl dimethicone (for example, KF-9908 and KF-9909, manufactured by Shin-Etsu Chemical Co., Ltd.). In particular, the component including at least one selected from triethoxycaprylylsilane, triethoxysilylethyl polydimethylsiloxyethyl dimethicone, triethoxysilylethyl polydimethylsiloxyethylhexyl dimethicone, and hydrogen dimethicone is preferable because such an agent can effect satisfactory hydrophobic treatment and ensures good dispersibility with the component (c). The hydrophobic treatment method is not particularly limited and may be any well-known method, with examples including wet treatment methods, dry treatment methods, and gas phase methods.

The surface-treated fine particle of titanium dioxide may be purchased from the market. For example, the surface-treated fine particle of titanium dioxide is commercially available under the trade names of MT-01, 02, 050OTS, 100Z, 100TV, 100SAS, 150EX, 200ST, 500SAM, 505SAS, 700Z, and 700BS (manufactured by TAYCA Co., Ltd.), ST-455, 455WS, 457ECS, 457SA, 495M, and 455FA (manufactured by Titan Kogyo, Ltd.), and STR-100A-LP, 100C-LP, 100W-LP, 100C-LF, and 40-LP (manufactured by Sakai Chemical Industry Co., Ltd.). Examples of the hydrophobized fine particle of titanium dioxides obtained by hydrophobizing hydrous silica-coated titanium dioxide particles include STR-100W-LP. It is preferable that the components (a) be not dispersions in which the particles are dispersed beforehand in an oil.

The number average primary particle diameter of the component (a) determined by image analysis of a transmission electron photomicrograph is 8 to 200 nm, and preferably 10 to 150 nm. If the number average primary particle diameter is more than 200 nm, the UV protection function is lowered and the powder applied leaves a white cast on the skin. If the particle diameter is less than 8 nm, dryness is increased and may adversely affect the feeling on use. The average primary particle diameter of the component (a) in the present invention is the average diameter of 200 particles measured by image analysis based on a transmission electron photomicrograph. When the powder is not spherical, the number average primary particle diameter is the average of the minor axe of the particles. The fine particle of titanium dioxide may be of spindle, needle, bunch, strip, substantial sphere, or rod shape.

The content of the component (a) is 10 to 70% by weight of the aqueous dispersion. From the point of view of usability, the content is preferably 10 to 65% by weight, more preferably 15 to 60% by weight, and still more preferably 20 to 55% by weight. If the content is less than 10% by weight, sufficient UV-shielding effects cannot be obtained. If the content is more than 70% by weight, there is a risk that spreadability on use is aggravated or the dispersion loses age stability and experiences a viscosity buildup.

[Components (b)]

The component (b) in the present invention is an aqueous component having at least two alcoholic hydroxyl groups, specifically, a component that dissolves in water at 25°C. Such components may be used singly, or two or more may be used in combination. Specific examples include sugar alcohols, such as Sorbitol (INCI), Maltose (INCI), Xylitol (INCI), Glucose (INCI), Glyceryl Glucoside (INCI), sodium chondroitin sulfate (labeling name, INCI: Sodium Chondroitin Sulfate), Methyl Gluceth-10 (INCI), Methyl Gluceth-20 (INCI), hyaluronic acid, phosphatidyl glycerol, and phosphatidyl inositol; glycols, such as BG (labeling name, INCI: Butylene Glycol), PG (labeling name, INCI: Propylene Glycol), DPG

(labeling name, INCI: Dipropylene Glycol), Pentylene Glycol (INCI), 1,10-Decanediol (INCI), Octanediol (INCI), and 1,2-Hexanediol (INCI); and polyhydric alcohols, such as Erythritol (INCI), Glycerin (INCI), Diglycerin (INCI), and polyethylene glycol. Of these, such glycols as BG (butylene glycol) and DPG (dipropylene glycol), and such polyhydric alcohols as glycerin are preferable because they dissolve in water in any proportion and also in view of the feeling on use and the versatility as cosmetic ingredients. In particular, glycols having two alcoholic hydroxyl groups in the molecule are more preferable.

The content of the component (b) is 1.0 to 30% by weight of the aqueous dispersion. The content is preferably 5 to 25% by weight, and more preferably 7 to 15% by weight from the point of view of water resistance. From the point of view of age stability, the content is more preferably 15 to 25% by weight, and particularly preferably 15 to 20% by weight. If the content is less than 1.0% by weight, the dispersion becomes less stable. If the content is more than 30% by weight, a cosmetic composition gives a sticky feeling on use, and the component (b) comes to prevent the component (c) described later from orienting toward the component (a), which may lead to a high viscosity or deterioration in the dispersibility in an aqueous medium. The component (b), when blended, helps the component (c) uniformly orient on the surface of the component (a).

[Components (c)]

The component (c) in the present invention is a polyglycerin-modified silicone that dissolves in the component (b). Such components may be used singly, or two or more may be used in combination. By the use of the polyglycerin-modified silicone that dissolves in the component (b), the polyglycerin-modified silicone can be blended in water and can function as a dispersant for the hydrophobized titanium dioxide fine particles in water. Regarding the phrase that the component (c) "dissolves in the component (b)", the component (c) "dissolves" when a mixture of 20% by weight of the component (c) with the component (b), after being allowed to stand at 25°C for 1 hour, is transparent to semi-transparent without any boundary, whereas the component (c) does "not dissolve" when the mixture becomes white turbid or separates into two layers. Being "transparent to semi-transparent" means that the total light transmission through a 1 cm thick cell filled with the mixture liquid is 50% or more as measured in accordance with JIS K7361-1: 1997. Those polyglycerin-modified silicones that do not dissolve in the component (b) do not belong to the components (c) in the present invention.

The chemical structure of the polyglycerin-modified silicone may be such that the silicone chain as the backbone is modified with a block or a graft of polyglycerin. In order to maintain uniform dispersibility of the component (a) in a cosmetic composition, it is preferable to use a branch-modified silicone. The silicone backbone may have a branched chain, such as a silicone chain. Specific examples include Polyglyceryl-3 Disiloxane Dimethicone (INCI). From the point of view of water resistance, the component (c) is preferably one that dissolves at 25°C when mixed in BG at a concentration of 20% by weight, and does not dissolve at 25°C when mixed in water at a concentration of 20% by weight. The polyglycerin-modified silicone in the present invention may be co-modified with hydrophilic groups other than polyglycerin groups, but 50% by weight or more of the hydrophilic groups are polyglycerin groups. From the point of view of water resistance, silicone exclusively modified with polyglycerin groups is preferable.

The polyglycerin-modified silicone used in the present invention preferably has an HLB (hydrophile-lipophile balance) value of 3 or more and less than 17, more preferably 5 or more and less than 15, and still more preferably 8 or more and less than 12. When the value is 3 or more, dispersibility is further enhanced. When the value is less than 17, water resistance is further enhanced. The HLB is calculated from "(sum of formula weights of hydrophilic group moieties/total molecular weight) $\times$ 20".

The content of the component (c) is 1.0 to 20% by weight of the aqueous dispersion, preferably 3 to 15% by weight, more preferably 4 to 10% by weight, and still more preferably 5 to 8% by weight. If the content is less than 1.0% by weight, the dispersion may become less stable. If the content is more than 20% by weight, molecules of the component (c) may be oriented on the component (a) that already has molecules of the component (c) oriented thereon, or molecules of the component (c) that have been oriented on the component (a) may be easily detached by other molecules of the component (c) not oriented on the component (a). Possible consequences are an increased viscosity of the dispersion and adverse effects on dispersibility in an aqueous medium.

Furthermore, the weight ratio (c)/(b) of the content of the component (c) to the content of the component (b) is preferably 0.2 to 0.9. From the points of view of dispersibility and water resistance, in particular, the ratio is more preferably 0.3 to 0.8, and still more preferably 0.4 to 0.5. By controlling the ratio to 0.2 or more, the component (c) can easily orient on the component (a) and the dispersion is prevented from a viscosity buildup and becomes more stable. On the other hand, controlling the ratio to 0.9 or less ensures that the component (c) is fully dispersed in the dispersion medium and can easily orient on the component (a), and the dispersion is prevented from a viscosity buildup and becomes more stable.

[Components (d)]

The component (d) in the present invention is water. Examples of water include ion exchanged water, distilled

water, deionized water, purified water defined in the Japanese Pharmacopoeia, spring water, and deep ocean water.

The content of the component (d) is 8 to 82% by weight of the aqueous dispersion, preferably 18 to 72% by weight, and more preferably 28 to 40% by weight. While a stable dispersion can be obtained even when the content is less than 8% by weight, such addition is difficult to attain the objective of lowering the viscosity by adding the component (d). If, on the other hand, the content is more than 82% by weight, a problem can arise in age stability.

The total content of the components (a), (b), (c), and (d) in the whole of the aqueous dispersion of the present invention is preferably 90 to 100% by weight, more preferably 95 to 100% by weight, and still more preferably 99 to 100% by weight. By controlling the total content of the components (a), (b), (c), and (d) to 90% by weight or more, the influence of components other than the components (a), (b), (c), and (d) can be reduced, and the dispersion thus prepared attains higher stability and a better feeling on use.

An antifoaming agent may be added to the aqueous dispersion to enhance handling properties at the time of preparation, filling, or the like. Examples of the antifoaming agents include Simethicone (INCI), Dimethicone (INCI), salts, such as sodium chloride (labeling name, INCI: Sodium Chloride), and polyether-modified silicones. Oils, such as dimethicone, may be used as previously emulsified emulsion types, and compounds, such as simethicone, may be used as self-emulsion types previously mixed with silicone surfactants or the like. Among those described above, in particular, simethicone and sodium chloride are preferable in terms of defoaming properties and antifoaming properties. Simethicone is most preferable in terms of the affinity for cosmetic ingredients. When the antifoaming agent is added, the amount thereof is preferably 0.0001 to 1% by weight of the whole of the aqueous dispersion, more preferably 0.0025 to 0.6% by weight, and still more preferably 0.005 to 0.01% by weight.

[Aqueous dispersions]

The aqueous dispersion of the present invention can benefit from facilitated blending in the aqueous medium. The term "dispersion" in the "aqueous dispersion" means that the mixture having a composition including 8 to 82% by weight of water shows temporary sedimentation when tested by the dispersibility test described later in Examples, but becomes uniformly dispersed upon continued stirring.

[Properties of the aqueous dispersions]

The dispersion of the present invention is preferably a liquid having a viscosity of 10 mPa·s or more and less than 200,000 mPa·s, more preferably 10 mPa·s or more and less than 10,000 mPa·s, and still more preferably 10 mPa·s or more and less than 5,000 mPa·s. By controlling the viscosity to 10 mPa·s or more, the stability of the dispersion is further enhanced. On the other hand, a viscosity of 200,000 mPa·s or less is preferable from the point of view of handling properties. The viscosity is measured at 25°C using a Brookfield viscometer (TVB-10, manufactured by Toki Sangyo Co., Ltd.) in accordance with the method described in JIS K7117-1: 1999.

[Methods for producing the aqueous dispersion]

The aqueous dispersion of the present invention may be prepared using any known method and apparatus without limitation. For example, use may be made of any agitators, mills, mixers, media agitating mills, planetary centrifugal agitators, and dispersing machines, such as Henschel mixers, ball mills, kneaders, planetary mixers, ribbon blenders, dispersing mixers, homo-mixers, jet mills, roll mills, bead mills, and high-pressure dispersers. From the point of view of mixing efficiency, in particular, it is preferable that the components be dispersed using a bead mill or a high-pressure disperser.

[Cosmetic compositions]

The aqueous dispersion of the present invention may be used in a variety of applications and, in particular, is usable as an ingredient for all cosmetic compositions that are externally applied to the skin and hair.

When the aqueous dispersion of the present invention is blended into a cosmetic composition, the amount thereof is preferably 0.5 to 60% by weight, more preferably 1.0 to 50% by weight, and still more preferably 5.0 to 40% by weight of the whole of the cosmetic composition. By controlling the amount to 0.5% by weight or more, sufficient UV protection effects can be expected. By controlling the amount to 60% by weight or less, the feeling on use is further enhanced.

The type of the cosmetic composition containing the aqueous dispersion of the present invention may be any of, for example, water-in-oil emulsion cosmetic, oil-in-water emulsion cosmetic, aqueous cosmetic, and multi-phase emulsions, such as W/O/W and O/W/O. Particularly when the dispersion is used in an oil-in-water emulsion cosmetic, the dispersion is easy to admit and the cosmetic composition that is obtained is stable and has excellent transparency, feeling

on use, and water resistance.

The form of the cosmetic composition of the present invention may be selected from among liquid, milky lotion, cream, solid, paste, gel, powder, pressed, multilayer, mousse, spray, stick, pencil, and other forms. The multilayer form refers to a cosmetic composition that separates into two or more layers on static holding. Such a cosmetic composition is placed in a container containing stainless steel balls or the like, and is used after shaking the container. Because the dispersion of the present invention has satisfactory dispersion properties, the cosmetic composition of such form can be readily re-dispersed. The chemical composition of such form is called shaking type or the like. Such a cosmetic composition gives a pleasant feeling on use because of easy stabilization, despite the need for shaking. Because the cosmetic composition of the present invention has satisfactory stability, it can also be used without separation into multiple layers. The spray form refers to a spraying cosmetic composition that is placed in a dispenser container, an aerosol container, or the like and is used by being sprayed through a nozzle. The cosmetic composition placed in a dispenser container is sprayed as mist through a nozzle on the dispenser. The cosmetic composition is placed in the aerosol container together with a spraying agent. The spraying agent is not particularly limited and may be selected from, for example, various liquefied petroleum gases (LPG), dimethyl ether, nitrogen gas, and carbon dioxide gas. The spraying agents may be used singly, or two or more may be used in appropriate combination. By virtue of its high dispersion properties, the aqueous dispersion of the present invention can be used even in this form of cosmetic composition.

The aqueous dispersion of the present invention is applicable to a variety of cosmetic compositions, particularly preferably to cosmetic compositions externally applied to the skin, such as skin care cosmetic compositions, make-up cosmetic compositions, antiperspirant cosmetic compositions, and UV-protecting cosmetic compositions, and cosmetic compositions externally applied to the hair, such as hair care cosmetic compositions. Exemplary skin care cosmetic compositions include toilet water, milky lotion, cream, cleansing agent, pack, oil liquid, massage cream, cosmetic liquid, cosmetic oil, detergent, deodorant, hand cream, lip cream, and anti-wrinkle agent. Exemplary make-up cosmetic compositions include make-up foundation, concealer, cosmetic powder, powder foundation, eye color, eye shadow, mascara, eye liner, eye brow cosmetic, and lip cosmetic. Exemplary antiperspirant cosmetic compositions include antiperspirant cosmetic compositions of roll-on, cream, solution, and stick types. Exemplary UV-protecting cosmetic compositions include sunscreen oil, sunscreen milky lotion, and sunscreen cream. Exemplary hair care cosmetic compositions include shampoo, conditioner, treatment, and setting agent. Among these, the UV-protecting cosmetic compositions are preferable.

The cosmetic composition of the present invention may contain various components that are commonly used in conventional cosmetics as long as the advantageous effects of the present invention are not impaired. Suitable components include, for example, (1) oils, (2) aqueous components other than the components (b) and (d), (3) surfactants other than the components (c), (4) powders other than the components (a), (5) compositions consisting of a crosslinked organopolysiloxane and an oil that is liquid at room temperature, (6) film-forming agents, and (7) other additives. These may be used singly, or two or more may be used in appropriate combination. The components contained in the dispersion described hereinabove may be added. However, the marked effects aimed at in the present invention cannot be obtained even if the components (a) to (d) of the present invention are added unless the dispersion of the present invention is added. The amounts of the components (a) to (d) not forming the dispersion are not particularly limited as long as the advantageous effects of the present invention are not impaired. When the components are added to the cosmetic composition separately from the dispersion, the amounts excluding the amounts in the dispersion are, for example, as follows. The amount of the component (a) is preferably 0.0 to 20% by weight, and more preferably 0.0 to 10% by weight of the cosmetic composition. The amount of the component (b) is preferably 0.0 to 70% by weight, more preferably 0.0 to 50% by weight, and still more preferably 0.0 to 25% by weight of the cosmetic composition. The amount of the component (c) is preferably 0.0 to 3.0% by weight, and more preferably 0.0 to 1.0% by weight of the cosmetic composition. The dispersion of the present invention is highly stable and can be added to a cosmetic composition without inhibiting the performance of an emulsifier or a dispersant in the cosmetic composition, thus allowing the amount of the component (c) other than in the dispersion to be reduced.

(1) Oils

The oils may be volatile or nonvolatile and may be solid, semisolid, or liquid at room temperature (25°C). Examples include silicone oils, silicone waxes, naturally occurring animal and plant oils and fats and semi-synthetic oils and fats, hydrocarbon oils, higher alcohols, fatty acids, ester oils, fluorinated oils, and UV absorbers.

• Silicone oils

Examples of the silicone oils include Dimethicone (INCI), Trisiloxane (INCI), alkyl-modified silicones, such as Methyl Trimethicone (INCI), Ethyl Trisiloxane (INCI), Ethyl Methicone (INCI), and Hexyl Dimethicone (INCI), long chain alkyl-modified silicones, such as Caprylyl Methicone (INCI), low to high viscosity linear or branched organopolysiloxanes,

such as Phenyl Trimethicone (INCI), Diphenyl Dimethicone (INCI), Diphenylsiloxyphenyl Trimethicone (INCI), Tetraphenyl Dimethyldisiloxane (INCI), and methyl hydrogen polysiloxane, cyclic organopolysiloxanes, such as Cyclotetrasiloxane (INCI), Cyclopentasiloxane (INCI), and Cyclohexasiloxane (INCI), amino-modified organopolysiloxanes, such as Amodimethicone (INCI) and Aminopropyl Dimethicone (INCI), pyrrolidone-modified organopolysiloxanes, such as PCA Dimethicone (INCI), antifoaming agents, such as Simethicone (INCI), pyrrolidone carboxylic acid-modified organopolysiloxanes, silicone rubbers, such as gum-like dimethylpolysiloxane having a high degree of polymerization, gum-like amino-modified organopolysiloxane, and gum-like dimethylsiloxane-methylphenylsiloxane copolymers, as well as low viscosity organopolysiloxane solutions of silicone gums and rubbers, amino acid-modified silicones, fluorine-modified silicones, silicone resins, and silicone resin solutions.

Commercially available examples of the silicone oils include KF-96L-1cs, KF-96L-1.5cs, KF-96L-2cs, KF-96A-6cs, KF-4422, KF-4418, KF-54, KF-54HV, KF-56A, and KF-995 manufactured by Shin-Etsu Chemical Co., Ltd.

• Solid oily components

When it is desired that the cosmetic composition of the present invention be solidified, an oily component that is solid at 25°C is preferably blended. Examples of the oily components that are solid at 25°C include waxes, hydrocarbons, esters, higher alcohols, and higher fatty acids having a melting point of preferably at least 40°C, more preferably 60 to 110°C. The oily component is not particularly limited as long as it is commonly blended in cosmetics. Specific examples include plant waxes, such as carnauba wax (INCI: Copernicia Cerifera (Carnauba) Wax), sugar cane wax, candelilla wax (INCI: Euphorbia Cerifera (Candelilla) Wax), purified candelilla wax, rice wax, Japan wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), insect wax, shellac wax, and sperm wax; semi-synthetic waxes, such as lanolin ester, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; wax esters, such as synthetic beeswax; amino acid stearyl alcohols, such as dioctyldodecyl lauroylglutamate, dioctyldodecyl lauroylglutamate, and dioctyldodecyl lauroylglutamate; fatty acids, such as stearic acid and behenic acid; silicone waxes, such as acrylate silicone resins in the form of acrylate silicone graft or block copolymers (such as acrylate silicone graft copolymer: KP-561P, manufactured by Shin-Etsu Chemical Co., Ltd.); and derivatives thereof. Any one, or two or more compounds selected from the foregoing are preferred.

• Natural animal and plant oils and semi-synthetic oils

Examples of the natural animal and plant oils and the semi-synthetic oils include naturally occurring plant oils, such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya California oil (labeling name, INCI: Torreya Californica (California Nutmeg) Oil), citronella grass oil (labeling name, INCI: Cymbopogon Nardus (Citronella) Oil), kaya seed oil (labeling name, INCI: Torreya Nucifera Seed Oil), kyounin oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), wheat germ oil (labeling name, INCI: Triticum Vulgare (Wheat) Germ Oil), rice germ oil (labeling name, INCI: Oryza Sativa (Rice) Germ Oil), rice bran oil (labeling name, INCI: Oryza Sativa (Rice) Bran Oil), camellia oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), gold tea oil (labeling name, INCI: Camellia Sinensis Seed Oil), camellia oil (labeling name, INCI: Camellia Japonica Seed Oil), evening primrose oil (labeling name, INCI: Oenothera Biennis (Evening Primrose) Oil), rapeseed oil (labeling name), germ oils, such as corn germ oil (labeling name, INCI: Zea Mays (Corn) Germ Oil) and wheat germ oil (labeling name, INCI: Triticum Vulgare (Wheat) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower seed oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Arachis Hypogaea (Peanut) Oil); natural animal oils, such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg yolk oil (labeling name, INCI: Egg Oil); and semi-synthetic oils and fats, such as hydrogenated coconut oil (labeling name, INCI: Hydrogenated Coconut Oil) and liquid lanolin (labeling name,

INCI: Lanolin Oil).

• Hydrocarbon oils

Examples of the hydrocarbon oils include linear or branched hydrocarbon oils. The hydrocarbon oils may be volatile or nonvolatile. Specific examples include Olefin Oligomers (INCI), isoparaffins, such as (C13,C14) Isoparaffin (INCI), Isododecane (INCI), Undecane (INCI), Tridecane (INCI), Dodecane (INCI), Isohexadecane (INCI), hydrogenated polyisobutene (labeling name, INCI: Hydrogenated Polyisobutene), Squalane (INCI), Mineral Oil (INCI), and alkanes, such as Coconut Alkanes (INCI) and (C13-15) Alkane (INCI).

• Higher alcohols

The higher alcohols include, for example, alcohols preferably having 6 or more carbon atoms, more preferably 10 to 30 carbon atoms. Specific examples of the higher alcohols include Lauryl Alcohol (INCI), Myristyl Alcohol (INCI), Palmityl Alcohol (INCI), Stearyl Alcohol (INCI), Behenyl Alcohol (INCI), Oleyl Alcohol (INCI), Isostearyl Alcohol (INCI), Octyl Dodecanol (INCI), Cholesterol (INCI), Phytosterol (INCI), and Batyl Alcohol (INCI).

• Ester oils

Examples of the ester oils include diisobutyl adipate (labeling name, INCI: Diisobutyl Adipate), dihexyldecyl adipate (labeling name), diheptylundecyl adipate (labeling name, INCI: Diheptylundecyl Adipate), n-alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and cocoalkyl (caprylate/caprate) (labeling name, INCI: Coco-Caprylate·Caprate).

Glyceride oils are also included in the ester oils, such as triethylhexanoin (labeling name, INCI:), glyceryl tri(caprylate/caprate) (labeling name, INCI: Caprylic/Capric Triglyceride), Cocoglyceryl (INCI), triglyceryl (caprylate/caprate/succinate) (labeling name, INCI: Caprylic/Capric/Succinic Triglyceride), and (caprylic/capric) glycerides (labeling name, INCI: Caprylic/Capric Glycerides).

• Fluorinated oils

Examples of the fluorinated oils include Perfluorodecalin (INCI), Perfluorononyl Dimethicone (INCI), and Perfluoromethylcyclopentane (INCI).

• UV absorbers

Suitable UV absorbers include oxybenzone-1 (labeling name, INCI: Benzophenone-1), oxybenzone-2 (labeling

name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethyl hexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methyl-bis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bisethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), Cinoxate (INCI), and ethylhexyl dimethoxybenzylidene dioxoimidazolidine propionate (labeling name, INCI: Ethylhexyl Dimethoxybenzylidene Dioxoimidazolidine Propionate).

(2) Aqueous components other than the components (b) and (d)

The aqueous components are not particularly limited as long as they do not belong to the components (b) or (d) and are commonly blended in cosmetics. Specific examples include humectants, such as Betaine (INCI), PCA-Na (labeling name, INCI: Sodium PCA), egg yolk lecithin, soybean lecithin, phosphatidylcholine, phosphatidylethanolamine, phosphatidylserine, and sphingophospholipid. Also included are water-soluble polymers, for example, gum Arabic, guar gum, carrageenan, agar, quince seed gum, locust bean gum, xanthan gum, pullulan, sodium carboxymethyl cellulose, hydroxyethyl cellulose, vinyl polymers, such as carboxyvinyl polymer, and acrylic polymers, such as (ammonium acryloyldimethyltaurate/vinylpyrrolidone) copolymer, (sodium acrylate/sodium acryloyldimethyltaurate) copolymer, (hydroxyethyl acrylate/sodium acryloyldimethyltaurate) copolymer, (acrylamide/sodium acryloyldimethyltaurate) copolymer, and polyacrylamide. Using acrylic polymers, an oil-in-water cosmetic composition can be stabilized in a relatively easy manner.

(3) Surfactants other than the components (c)

The surfactants may be nonionic, anionic, cationic, or ampholytic. The surfactants are not particularly limited as long as they do not belong to the components (c) and are commonly blended in cosmetics. Of the surfactants, one, or two or more surfactants selected from non-crosslinked silicone surfactants and crosslinked silicone surfactants are preferred because a stable cosmetic composition is obtainable. In any cases where the surfactant is blended, the amount thereof is preferably 0.1 to 20% by weight of the overall cosmetic composition. An amount of at least 0.1% by weight is preferred in that the dispersing and emulsifying functions are fully achieved whereas an amount of up to 20% by weight is preferred because the risk that the cosmetic composition gives a sticky feeling on use is eliminated. The surfactant preferably has an HLB of 2 to 14.5, though not limitative, for the purpose of maintaining the cosmetic composition water resistant.

The non-crosslinked silicone surfactants are linear or branched silicones in which some methyl groups on the backbone are substituted with hydrophilic groups, such as polyethylene glycol and polyglycerin. Specifically, preferred such surfactants are linear or branched polyoxyethylene-modified organopolysiloxanes, linear or branched polyoxyethylene polyoxypropylene-modified organopolysiloxanes, linear or branched polyoxyethylene·alkyl-co-modified organopolysiloxanes, linear or branched polyoxyethylene polyoxypropylene·alkyl-co-modified organopolysiloxanes, linear or branched polyglycerin-modified organopolysiloxanes, linear or branched polyglycerin·alkyl-co-modified organopolysiloxanes, and linear or branched pyrrolidone-modified organopolysiloxanes. Included are PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Polydimethylsiloxyethyl Dimethicone (INCI), Lauryl Polyglyceryl-3 Polydimethylsiloxyethyl Dimethicone (INCI), and Isostearyl Polyglyceryl-3 Dimethicone (INCI).

Commercially available examples include KF-6011, KF-6011P, KF-6012, KF-6015, KF-6017, KF-6043, KF-6028, KF-6038, KF-6048, KF-6104, KF-6106, KF-6105, KF-6180, and KF-6115, manufactured by Shin-Etsu Chemical Co., Ltd.

Examples of the crosslinked silicone surfactants include (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), (PEG-15/Lauryl Polydimethylsiloxyethyl Dimethicone) Crosspolymer (INCI), (Dimethicone/Polyglycerin-3) Crosspolymer (INCI), (Lauryl

Dimethicone/Polyglycerin-3) Crosspolymer (INCI), and (Polyglycerin-3/Lauryl Polydimethylsiloxyethyl Dimethicone) Crosspolymer (INCI). On use of the crosslinked silicone surfactant, it is preferred that in a composition consisting of the crosslinked silicone surfactant and an oil that is liquid at room temperature, the crosslinked silicone surfactant swell with the liquid oil as a result of being impregnated with an amount of more than its own weight of the liquid oil.

Suitable liquid oils include liquid silicone oils, hydrocarbon oils, ester oils, natural animal and plant oils, semi-synthetic oils, and fluorinated oils, all exemplified above as the oils (1) that are optional components. Illustrative examples include Cyclopentasiloxane (INCI), Dimethicone (INCI), Caprylyl Methicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Commercially available examples of the crosslinked silicone surfactants that are swollen with the liquid oil include KSG-210, KSG-240, KSG-270, KSG-310, KSG-320, KSG-330, KSG-340, KSG-320Z, KSG-350Z, KSG-710, KSG-790, KSG-810, KSG-820, KSG-830, KSG-840, KSG-820Z, and KSG-850Z from Shin-Etsu Chemical Co., Ltd.

(4) Powders other than the components (a)

Suitable powders other than the components (a) include coloring pigments, inorganic powders, metal powders, organic powders, and inorganic/organic composite powders, which are exemplified below.

• Coloring pigments

The coloring pigments are not particularly limited as long as they are commonly used in cosmetics for the purpose of coloring. Examples include red iron oxide (labeling name, INCI: Iron Oxides), yellow iron oxide (labeling name, INCI: Iron Oxides), white titanium dioxide (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium dioxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), cobalt titanate (labeling name, INCI: Cobalt Titanium dioxide), fired titanium/titanium dioxide (labeling name, INCI: Titanium/Titanium Dioxide), Li/cobalt titanate (labeling name, INCI: Lithium Cobalt Titanate), cobalt titanate (labeling name, INCI: Cobalt Titanium dioxide), sintered iron oxide/titanium dioxide (labeling name), composites doped with a different metal, such as iron oxide-doped titanium dioxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as $\gamma$-iron oxide, inorganic yellow pigments, such as loess, and colored pigments, such as lake form of tar dyes and lake form of natural dyes. Any of the foregoing may be used.

The pigment may take any shape including spherical, generally spherical, bar, spindle, petal, strip, and irregular shapes. Its geometry is not particularly limited as long as a color can be imparted to the cosmetic composition.

• Inorganic powders

Examples of the inorganic powders include microparticles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), Talc (INCI), Mica (INCI), Kaolin (INCI), synthetic fluorophlogopite (labeling name, INCI: Synthetic Fluorphlogopite), synthetic ferrous golden mica (labeling name), black mica (labeling name, INCI: Biotite), potassium silicate (labeling name, INCI: Potassium Silicate), Silica (INCI), aluminum silicate (labeling name, INCI: Aluminum Silicate), magnesium silicate (labeling name, INCI: Magnesium Silicate), Al/Mg silicate (labeling name, INCI: Magnesium Aluminum Silicate), calcium silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), Hydroxyapatite (INCI), Bentonite (INCI), Montmorillonite (INCI), Hectorite (INCI), Zeolite (INCI), Alumina (INCI), Al hydroxide (labeling name, INCI: Aluminum Hydroxide), boron nitride (labeling name, INCI: Boron Nitride), and glass (labeling name, INCI: Glass).

Also, examples of the inorganic coloring pearly pigments include pearly agents, such as Mica (INCI) coated with titanium dioxide (labeling name, INCI: Titanium dioxide), and synthetic fluorophlogopite (labeling name, INCI: Synthetic Fluorphlogopite) coated with titanium dioxide (labeling name, INCI: Titanium Dioxide); and pearly pigments, such as bismuth oxychloride (labeling name, INCI: Bismuth Oxychloride), bismuth oxychloride (labeling name, INCI: Bismuth

Oxychloride) coated with titanium dioxide (labeling name, INCI: Titanium Dioxide), Talc (INCI) coated with titanium dioxide (labeling name, INCI: Titanium Dioxide), fish scale flakes (labeling name), and coloring mica coated with titanium dioxide (labeling name, INCI: Titanium Dioxide). These may either be untreated or have undergone well-known surface treatment commonly used for cosmetics.

• Metal powders

Examples of the metal powders include metal microparticles, such as aluminum (labeling name, INCI: Aluminum, Aluminum Powder), copper (labeling name, INCI: Copper Powder), silver (labeling name, INCI: Silver Powder), and gold (labeling name, INCI: Gold).

• Organic powders

Examples of the organic powders include powders of silicones, polyamides, polyacrylic acid-acrylate, polyesters, Polyethylene (INCI), Polypropylene (INCI), Polystyrene (INCI), styrene-acrylic acid copolymers, divinylbenzene-styrene copolymers, polyurethane, vinyl resins, urea resins, melamine resins, benzoguanamine, polymethyl benzoguanamine, tetrafluoroethylene, polymethyl methacrylate, Cellulose (INCI), Silk (INCI), nylon (labeling name), phenolic resins, epoxy resins, and polycarbonate.

In particular, examples of the silicones include silicone resin particles; Polymethylsilsesquioxane (INCI), silicone rubber powder, silicone resin-coated silicone rubber powder; (vinyl dimethicone/methicone silsesquioxane) crosspolymer (labeling name, INCI: Vinyl Dimethicone/Methicone Silsesquioxane Crosspolymer), (diphenyl dimethicone/vinyldiphenyl dimethicone/silsesquioxane) crosspolymer (labeling name, INCI: Diphenyl Dimethicone/Vinyl Diphenyl Dimethicone/-Silsesquioxane Crosspolymer), Polysilicone-1 Crosspolymer (INCI), and Polysilicone-22 (INCI).

Commercially available examples of the silicone powders include KMP-590, KMP-591, KMP-592, KMP-597, KMP-598, KSP-100, KSP-101, KSP-102, KSP-105, KSP-100W, KSP-300, KSP-411, KSP-441, KM-9729, and KM-440, from Shin-Etsu Chemical Co., Ltd.

Examples further include metal soaps. Specific examples include powders of zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), Zn/Na cetylphosphate (labeling name, INCI: Sodium Zinc Cetyl Phosphate), and K cetylphosphate (labeling name, INCI: Potassium Cetyl Phosphate).

Examples further include organic colorants. Specific examples include tar dyes, such as Red #3, Red #104 (1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230 (1) (labeling name, INCI: Red 22, Red 22 Lake), Red #230 (2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202 (1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 (labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), and Orange #207 (labeling name, INCI: Orange 11); and natural dyes, such as Cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

• Inorganic/organic composite powders

Exemplary of the inorganic/organic composite powders is a composite powder obtained by coating an inorganic powder on the surface with an organic powder by any well-known method.

The powders described above may be used as having been treated on the surface of particles. The surface treating agent is preferably one capable of imparting hydrophobicity from the aspect of water resistance of the cosmetic composition. The hydrophobizing agent is not particularly limited. Exemplary treating agents include silicone treating agents, waxes, paraffins, organic fluorine compounds, such as perfluoroalkyl phosphate salts, surfactants, amino acids, such as N-acylglutamic acid, and metal soaps, such as aluminum stearate and magnesium myristate.

Among others, the silicone treating agents are preferably used. Included are silanes or silylating agents, such as

Triethoxycaprylylsilane (INCI); Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and acrylates/tridecyl acrylate/triethoxysilylpropyl methacrylate/dimethicone methacrylate copolymer (labeling name, INCI: Acrylates/Tridecyl Acrylate/Triethoxysilylpropyl Methacrylate/Dimethicone Methacrylate Copolymer). Specific examples of the silicone treating agents include AES-3083, KF-99P, KF-9901, KF-9908, KF-9909, KP-574, and KP-541, from Shin-Etsu Chemical Co., Ltd.

The surface hydrophobizing agents may be used singly, or two or more may be used in combination. Specific examples of the surface treated coloring pigments include KTP-09 series, especially KTP-09W, KTP-09R, KTP-09Y, and KTP-09B, from Shin-Etsu Chemical Co., Ltd.

• UV absorbing/scattering agents

In addition to the dispersion of the present invention, a dispersion having the component (a), namely, UV absorbing/scattering particles, previously dispersed in an oil may also be used.

Suitable oils include liquid silicone oils, hydrocarbon oils, ester oils, natural animal and plant oils, semi-synthetic oils, and fluorinated oils, all exemplified above as the oils (1) that are optional components.

Specific examples of the dispersions having UV absorbing/scattering particles previously dispersed in an oil include SPD series (product name), especially SPD-T5, SPD-T5L, SPD-Z5, SPD-T6, SPD-Z6, SPD-T7, and SPD-Z7L, from Shin-Etsu Chemical Co., Ltd.

(5) Compositions consisting of a crosslinked organopolysiloxane and an oil that is liquid at room temperature

In the composition consisting of a crosslinked organopolysiloxane and an oil that is liquid at room temperature, it is preferred that the crosslinked organopolysiloxane swell with the liquid oil as a result of being impregnated with an amount of more than its own weight of the liquid oil. Suitable liquid oils include liquid silicone oils, hydrocarbon oils, ester oils, natural animal and plant oils, semi-synthetic oils, and fluorinated oils, all exemplified above as the oils (1) that are optional components. Illustrative examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), Squalane (INCI), and cocoalkyl (caprylate/caprate) (labeling name, INCI: Coco-Caprylate·Caprate).

Unlike the crosslinked silicone surfactants as the components (3) mentioned above, the components (5) are compounds having neither polyether nor polyglycerin structure in the molecular structure. Specific examples include Dimethicone/Vinyl Dimethicone Crosspolymer (INCI), dimethicone/phenyl vinyl dimethicone crosspolymer (labeling name, INCI: Dimethicone/Phenyl Vinyl Dimethicone Crosspolymer), Vinyl Dimethicone/Lauryl Dimethicone Crosspolymer (INCI), and Lauryl Polydimethylsiloxyethyl Dimethicone/Bis-Vinyl Dimethicone Crosspolymer (INCI).

Commercially available examples of the compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-45, KSG-042Z, KSG-045Z, KSG-048Z, and KM-116, from Shin-Etsu Chemical Co., Ltd.

(6) Film-forming agents

The film-forming agent is blended mainly for the purpose of further maintaining the effect-sustainability of the cosmetic composition. A silicone-based agent is preferred from the aspect of imparting water repellency, though the agent is not limited thereto. Specifically, use may be made of, for example, trimethylsiloxysilicic acid, acrylic-silicone film formers, silicone-modified norbornene, silicone-modified pullulan, and silicone-modified polyvinyl alcohol.

Examples of the film-forming agents in the form of silicone-based agent include trimethylsiloxysilicic acid (labeling name, INCI: Trimethylsiloxysilicate), Acrylates/Dimethicone Copolymer (INCI), Norbornene/Tris-(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan).

The film-forming agent may be previously dissolved in an oil that is liquid at room temperature before it is blended in the cosmetic composition. Suitable liquid oils used herein include liquid silicone oils, hydrocarbon oils, ester oils, natural animal and plant oils, semi-synthetic oils, and fluorinated oils, all exemplified above as the oils (1) that are optional components. Commercially available examples of the silicone film-forming agents include KF-7312J, KP-545, KP-549, KP-543, NBN-30-ID, TSPL-30-ID, and TSPL-30-D5, from Shin-Etsu Chemical Co., Ltd.

(7) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, and clathrate compounds.

• Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid ) and $\alpha,\gamma$-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals, such as Disteardimonium Hectorite (INCI), Stearalkonium Hectorite (INCI), and hectorite; and Stearalkonium Bentonite (INCI).

• Preservatives/antibacterial agents

Suitable preservatives and antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, bisabolol, ethylhexylglycerin, glyceryl caprylate, caprylhydroxaminc acid, hexyl dimethylolpropanoate, polyaminopropyl biguanide, photosensitizers, silver, and plant extracts. Preservatives may be used singly, or two or more may be used in combination. In particular, polylysine, bisabolol, ethylhexylglycerin, glyceryl caprylate, caprylhydroxamic acid, hexyl dimethylolpropanoate, polyaminopropyl biguanide, ethylhexylglycerin, and phenoxyethanol are preferable because they are easily blended into the cosmetic composition and are expected to produce preservative effects. Ethylhexylglycerin, phenoxyethanol, and glyceryl caprylate are particularly preferable in terms of the compatibility with the dispersion of the present invention. The addition of preservatives is expected to suppress bacterial contamination and enhance the storage stability of the dispersion.

• Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, aluminum potassium sulfate, aluminum zirconium tetrachlorohydrate, and aluminum zirconium trichlorohydrex glycine. The preferred components that exert a higher effect include aluminum halohydrates, aluminum halides, and complexes or mixtures thereof with zirconium oxyhalides and zirconium hydroxyhalides (e.g., aluminum zirconium tetrachlorohydrate and aluminum zirconium trichlorohydrex glycine.

• Fragrances

Examples of the fragrances include natural fragrances and synthetic fragrances. Suitable natural fragrances include plant fragrances separated from flowers, leaves, stems, pericarps, and the like; and animal fragrances, such as musk and civet. Suitable synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

• Salts

Examples of the salts include inorganic salts, organic acid salts, amine salts, and amino acid salts. Exemplary inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanol amine, and salts of amino acids, such as glutamic acid. Also useful are salts of hyaluronic acid, chondroitin sulfate, aluminum zirconium glycine complex, and acid-alkali neutral salts used in cosmetic formulations. In particular, sodium

chloride is preferred from the aspects of dissolution, feeling on use, and anti-foaming. When used in large amounts, the salt can inhibit the water-soluble polymer from swelling.

• Antioxidants

Examples of the antioxidants include, but are not particularly limited to, carotenoid, ascorbic acid and salts thereof, ascorbyl stearate, tocophenol, tocophenol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

• Acidity regulators

Suitable acidity regulators include lactic acid, citric acid, glycolic acid, succinic acid, tartaric acid, dl-malic acid, potassium carbonate, sodium hydrogencarbonate, and ammonium hydrogencarbonate.

• Chelating agents

Suitable chelating agents include alanine, sodium EDTA, sodium polyphosphate, sodium metaphosphate, and phosphoric acid.

• Refreshing agents

Suitable refreshing agents include L-menthol, camphor, and menthyl lactate.

• Anti-inflammatory agents

Suitable anti-inflammatory agents include allantoin, glycyrrhizinic acid and salts thereof, glycyrrhetinic acid, stearyl glycyrrhetinate, tranexamic acid, and azulene.

• Skin modifying ingredients

Suitable skin modifying ingredients include brightening or whitening agents, such as placenta extract, arbutin, glutathione, and Saxifraga Sarmentosa Extract; cell activators, such as royal jelly, photosensitizers, cholesterol derivatives, and bovine blood extracts; anti-skin-roughening agents; blood flow enhancing agents, such as vanillylamide nonanoate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

• Vitamins

Suitable vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

• Amino acids

Suitable amino acids include glycine, valine, leucine, isoleucine, serine, threonine, phenylalanine, arginine, lysine, aspartic acid, glutamic acid, cystine, cysteine, methionine, and tryptophan.

• Nucleic acids

Typical of the nucleic acids is deoxyribonucleic acid.

• Hormones

Suitable hormones include estradiol and ethenyl estradiol.

• Clathrate compounds

Typical of the clathrate compounds is cyclodextrin.

EXAMPLES

Production Examples, Examples and Comparative Examples are shown below by way of illustration of the present invention and not by way of limitation. In the examples below, the compositional percent (%) and the ratio are by weight unless otherwise stated. The amount of product name is the amount of the product blended. The order in which the siloxane units in parentheses in the formula are bonded is not limited to the illustration.

(Production Example 1)

A reactor was charged with methyl hydrogen polysiloxane (H-1) and polyglycerin monoallyl ether (G-1) in a molar ratio of 1:4, and was further charged with isopropyl alcohol and a platinum catalyst. The reaction was carried out at 80°C. The isopropyl alcohol was then removed by heating under reduced pressure. A polyglycerin-modified silicone (A-1) was thus obtained. In (A-1), the hydrophile-lipophile balance (HLB) value defined as "(sum of molecular weights of hydrophilic group moieties/total molecular weight) × 20" is 8.

(H-1)

[Chem. 1]

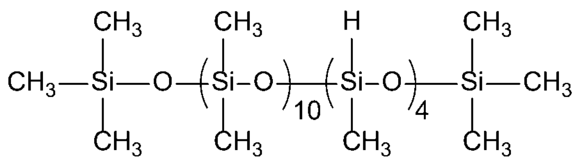

(G-1)

[Chem. 2]

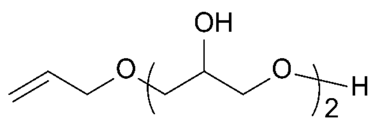

(A-1)

[Chem. 3]

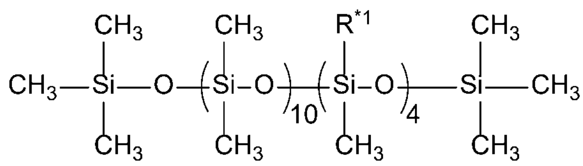

$R^{*1}$: $-C_3H_6O(CH_2CH(OH)CH_2O)_2H$

(Production Example 2)

A reactor was charged with methyl hydrogen polysiloxane (H-2) and polyglycerin monoallyl ether (G-2) in a molar ratio of 1:1, and was further charged with isopropyl alcohol and a platinum catalyst. The reaction was carried out at 80°C. The isopropyl alcohol was removed by heating under reduced pressure. A polyglycerin-modified silicone (A-2) was thus obtained. In (A-2), the hydrophile-lipophile balance (HLB) value defined as "(sum of molecular weights of hydrophilic group moieties/total molecular weight) × 20" is 12.

(H-2)

[Chem. 4]

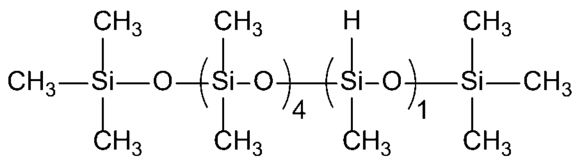

(G-2)

[Chem. 5]

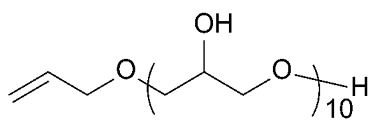

(A-2)

[Chem. 6]

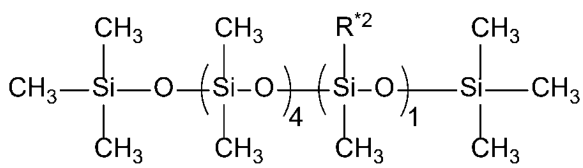

$R^{*2}$ $-C_3H_6O(CH_2CH(OH)CH_2O)_{10}H$

(Production Example 3)

A reactor was charged with methyl hydrogen polysiloxane (H-3) and polyglycerin monoallyl ether (G-3) in a molar ratio of 1:2, and was further charged with isopropyl alcohol and a platinum catalyst. The reaction was carried out at 80°C. The isopropyl alcohol was removed by heating under reduced pressure. A polyglycerin-modified silicone (A-3) was thus obtained. In (A-3), the hydrophile-lipophile balance (HLB) value defined as "(sum of molecular weights of hydrophilic group moieties/total molecular weight) × 20" is 10.

(H-3)

[Chem. 7]

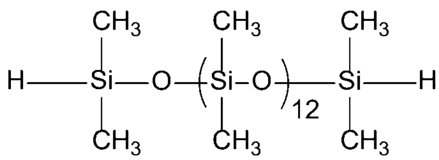

(G-3)

[Chem. 8]

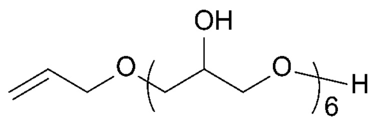

(A-3)

[Chem. 9]

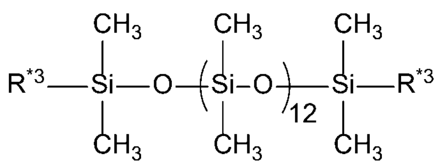

$R^{*3}$: $-C_3H_6O(CH_2CH(OH)CH_2O)_6H$

(Production Example 4)

A reactor was charged with methyl hydrogen polysiloxane (H-4) and polyglycerin diallyl ether (G-4) in a molar ratio of 2:1, and was further charged with isopropyl alcohol and a platinum catalyst. The reaction was carried out at 80°C. The isopropyl alcohol was removed by heating under reduced pressure. A polyglycerin-modified silicone (A-4) was thus obtained. In (A-4), the hydrophile-lipophile balance (HLB) value defined as "(sum of molecular weights of hydrophilic group moieties/total molecular weight) × 20" is 10.

(H-4)

[Chem. 10]

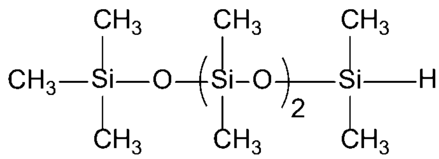

(G-4)

[Chem. 11]

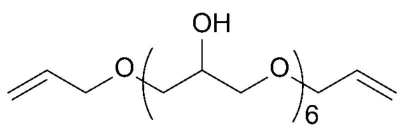

(A-4)

[Chem. 12]

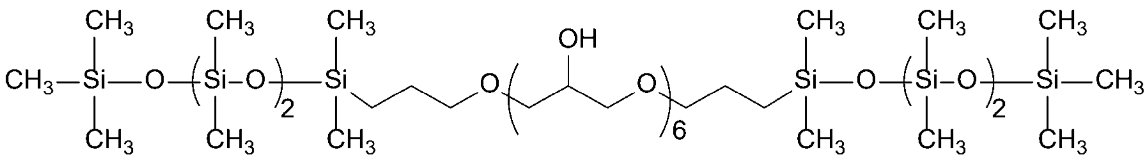

(Comparative Production Example 1)

A reactor was charged with methyl hydrogen polysiloxane (H-5), polyglycerin monoallyl ether (G-5), and polyoxyalkylene monoallyl ether (E-1) in a molar ratio of 1:1:2.7, and was further charged with a platinum catalyst. The reaction was carried out at 80°C. Subsequently, isopropyl alcohol was removed by heating under reduced pressure. An organopolysiloxane (A-5) of Comparative Production Example 1 having polyglycerin groups and polyoxyalkylene groups was thus obtained. In (A-5), the hydrophile-lipophile balance (HLB) value defined as "(sum of molecular weights of hydrophilic group moieties/total molecular weight) × 20" is 10. In the organopolysiloxane (A-5) of Comparative Production Example 1 having polyglycerin groups and polyoxyalkylene groups, 20% by weight of the hydrophilic groups are polyglycerin groups.

(H-5)

[Chem. 13]

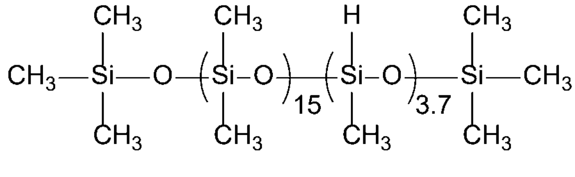

(G-5)

[Chem. 14]

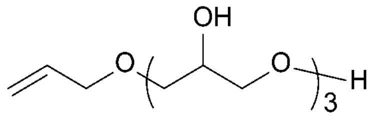

(E-1)

[Chem. 15]

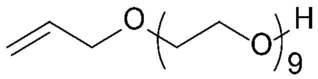

(A-5)

[Chem. 16]

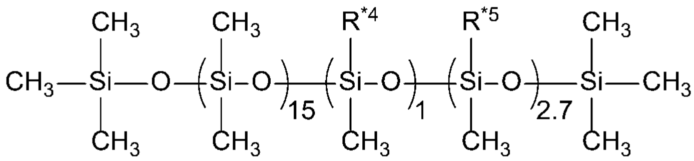

$R^{*4}$: $-C_3H_6O(CH_2CH(OH)CH_2O)_3H$

$R^{*5}$: $-C_3H_6O(C_2H_4O)_9H$

[Whether or not aqueous dispersants dissolve]

The components described below were each mixed with water or BG so that their concentration would be 20%, and then allowed to stand at 25°C for 1 hour. The rating was "dissolve" when the mixture was transparent to semi-transparent without any boundary. The rating was "not dissolve" when the mixture had become white turbid or had separated into two layers. Being "transparent to semi-transparent" means that the total light transmission through a 1 cm thick cell filled with the mixture liquid is 50% or more as measured in accordance with JIS K7361-1: 1997.

[Table 1]

| | Solubility in water | Solubility in BG |
|---|---|---|
| (c) Polyglyceryl-3 disiloxane dimethicone (HLB 10) | Not dissolve | Dissolve |
| (c) Polyglyceryl-3 disiloxane dimethicone (HLB 12) | Dissolve | Dissolve |
| Polyglyceryl-3 polydimethylsiloxyethyl dimethicone (HLB 1) | Not dissolve | Not dissolve |
| (c) Production Example 1 | Not dissolve | Dissolve |
| (c) Production Example 2 | Dissolve | Dissolve |
| (c) Production Example 3 | Not dissolve | Dissolve |
| (c) Production Example 4 | Dissolve | Dissolve |
| PEG-9 dimethicone | Dissolve | Dissolve |

[Examples]

Aqueous dispersions were obtained by preparing slurries according to the formulations described in Tables 2 to 4

below using a bead mill. The aqueous dispersions obtained were evaluated for dispersion properties according to the evaluation criteria described below. The results are also described in the tables.

[Success or failure of the preparation of the aqueous dispersion]

A: The preparation of dispersion was successful (the dispersion had temporary sedimentation, but was uniformly dispersed upon continued stirring).
D: The preparation of dispersion failed.

The sample was judged "Pass" when rated with "A".

[Evaluation of long-term stability]

75 g of the aqueous dispersion was placed into a 50 mL container and stored in a thermostatic chamber at 25°C for one month. The long-term stability was evaluated according to the evaluation criteria below. The results were judged according to the criterion below.

[Judgement criteria]

$$\text{Viscosity change (\%)} = [\{(\text{initial viscosity}) - (\text{viscosity after one month})\}/(\text{initial viscosity})] \times 100$$

The judgement criteria are as follows.

A: The viscosity change after one month is less than 20%.
B: The viscosity change after one month is 20% or more and less than 30%.
C: The viscosity change after one month is 30% or more.
D: Floating matter is present in the upper layer.

The sample was judged "Pass" when rated with "C" or above.

[Evaluation of water resistance]

The aqueous dispersions were evaluated for water resistance on application according to the evaluation criteria below. The results were judged according to the criterion below.

[Judgement criteria]

A: Remains after exposed to running water for 1 minute or more.
B: Remains after exposed to running water for 30 seconds or more and less than 1 minute.
C: Vanishes after exposed to running water for 10 seconds or more and less than 30 seconds.
D: Vanishes after exposed to running water for less than 10 seconds.

The sample was judged "Pass" when rated with "C" or above.

[Evaluation of feeling on use]

The sample was evaluated for the feeling on use (or non-stickiness) on application by a panel of 10 professional members according to the evaluation criteria below. The results of the 10 members were averaged, and the feeling on use was rated according to the judgment criteria below.

[Evaluation criteria]

5 points: excellent
4 points: good
3 points: moderate
2 points: rather poor
1 point: poor

[Judgment criteria]

A: The average point is 4.5 points or more.
B: The average point is 3.5 points or more and less than 4.5 points.
C: The average point is 2.5 points or more and less than 3.5 points.
D: The average point is less than 2.5 points.

The sample was judged "Pass" when rated with "C" or above.

[Table 2]

| Formulation (%) | Example | | | | | | | | | |
|---|---|---|---|---|---|---|---|---|---|---|
| | 1 | 2 | 3 | 4 | 5 | 6 | 7 | 8 | 9 | 10 |
| (a) STR-100W-LP (Note 1) | 50 | 50 | 50 | 50 | 50 | 50 | 45 | 50 | 50 | 50 |
| (b) BG | 10 | 10 | 10 | 13 | | | 13 | 7 | 15 | 20 |
| (b) DPG | | | | | 13 | | | | | |
| (b) Glycerin | | | | | | 13 | | | | |
| (c) Polyglyceryl-3 disiloxane dimethicone (HLB 10) | 8 | 7 | 6 | 6 | 6 | 6 | | 10 | 6 | 6 |
| (c) Polyglyceryl-3 disiloxane dimethicone (HLB 12) | | | | | | | 6 | | | |
| (d) Water | 32 | 33 | 34 | 31 | 31 | 31 | 36 | 33 | 29 | 24 |
| Total | 100 | 100 | 100 | 100 | 100 | 100 | 100 | 100 | 100 | 100 |
| (c)/(b) | 0.80 | 0.70 | 0.60 | 0.46 | 0.46 | 0.46 | 0.46 | 1.43 | 0.40 | 0.30 |
| Success or failure of preparation | A | A | A | A | A | A | A | A | A | A |
| Long-term stability | C | C | C | B | C | B | B | C | A | A |
| Water resistance | A | A | A | A | A | B | C | A | A | B |
| Feeling on use | C | B | B | A | A | B | A | C | A | B |

[Table 3]

| Formulation (%) | Example | | | |
|---|---|---|---|---|
| | 11 | 12 | 13 | 14 |
| (a) STR-100W-LP (Note 1) | 50 | 50 | 50 | 50 |
| (b) BG | 13 | 13 | 13 | 13 |
| (c) Production Example 1 (HLB 8) | 6 | | | |
| (c) Production Example 2 (HLB 12) | | 6 | | |

(continued)

| Formulation (%) | Example | | | |
|---|---|---|---|---|
| | 11 | 12 | 13 | 14 |
| (c) Production Example 3 (HLB 10) | | | 6 | |
| (c) Production Example 4 (HLB 10) | | | | 6 |
| (d) Water | 31 | 31 | 31 | 31 |
| Total | 100 | 100 | 100 | 100 |
| (c)/(b) | 0.46 | 0.46 | 0.46 | 0.46 |
| Success or failure of preparation | A | A | A | A |
| Long-term stability | C | A | A | A |
| Water resistance | A | C | B | B |
| Feeling on use | A | B | C | B |

**[Table** 4]

| Formulation (%) | Comparative Example | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| (a) STR-100W-LP (Note 1) | | | 80 | 50 | 50 | 50 | 50 | 50 | 50 | 50 | 50 | 50 |
| Stearic acid-treated titanium dioxide (Note 2) | 50 | | | | | | | | | | | |
| Hydrous silica-treated titanium dioxide (Note 3) | | 30 | | | | | | | | | | |
| (b) BG | 10 | 10 | 10 | | 10 | 13 | 10 | 13 | 10 | 10 | 10 | 20 |
| (c) Polyglyceryl-3 disiloxane dimethicone (HLB 10) | 7 | 5 | 7 | 7 | | | | | | | | |
| Polyglyceryl-3 polydimethylsiloxyethyl dimethicone (HLB 1) | | | | | | 7 | | | | | | |
| Comparative Production Example 1 | | | | | | | | | | | | 6 |
| PEG-9 dimethicone | | | | | | | 7 | 12 | | | | |
| Amodimethicone | | | | | | | | | 7 | | | |
| Aminopropyl dimethicone | | | | | | | | | | 7 | | |
| Carboxydecyl dimethicone | | | | | | | | | | | 7 | |
| (d) Water | 33 | 55 | 3 | 43 | 40 | 30 | 33 | 25 | 33 | 33 | 33 | 24 |
| Total | 100 | 100 | 100 | 100 | 100 | 100 | 100 | 100 | 100 | 100 | 100 | 100 |
| (c)/(b) | 0.70 | 0.50 | 0.70 | 0.00 | 0.00 | 0.00 | 0.00 | 0.00 | 0.00 | 0.00 | 0.00 | 0.00 |
| Success or failure of preparation | A | A | D | D | D | D | D | A | D | D | D | A |
| Long-term stability | D | D | - | - | - | - | - | B | - | - | - | A |
| Water resistance | A | D | - | - | - | - | - | D | - | - | - | D |

(continued)

| Formulation (%) | Comparative Example | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Feeling on use | A | D | - | - | - | - | - | D | - | - | - | B |

Notes in Tables 2 to 4 are as follows:

(Note 1) Fine particle of titanium dioxide (labeling name, INCI: Titanium Dioxide) coated with hydrous silica (labeling name, INCI: Hydrated Silica) and further treated with hydrogen dimethicone (labeling name, INCI: Hydrogen Dimethicone)
Number average primary particle diameter determined by image analysis of a transmission electron photomicrograph: 15 nm (average diameter of 200 particles measured by image analysis of a transmission electron photomicrograph)

(Note 2) Fine particle of titanium dioxide (labeling name, INCI: Titanium Dioxide) treated with stearic acid (labeling name, INCI: Stearic Acid)

(Note 3) Fine particle of titanium dioxide (labeling name, INCI: Titanium Dioxide) coated with hydrous silica (labeling name, INCI: Hydrated Silica)

As clear from Tables 2 and 3, the aqueous dispersions (slurries) obtained in Examples of the present invention achieved good dispersion properties in the aqueous medium. In contract, Comparative Example 1 that did not involve the component (a) failed to prepare a dispersion. Comparative Example 2 gave a dispersion but was unsatisfactory in long-term stability, water resistance, and feeling on use. Dispersions could not be prepared in Comparative Example 4 that did not involve the component (b), Comparative Example 3 that involved 70% more of the component (a), and Comparative Examples 5 to 7 and 9 to 11 that did not involve the component (c). Comparative Example 8 gave a dispersion but was unsatisfactory in water resistance and feeling on use. Comparative Example 12 gave a dispersion but was poor in water resistance.
The number average primary particle diameters of the fine particle of titanium dioxides used in the following Examples were in the range of 8 to 200 nm.

[Example 15 and Comparative Example 13]

O/W creams were prepared according to the formulations described in Table 5 below.

[Production method]

[Example 15]

A: Components 5 to 12 were mixed.
B: Component 4 was added to the composition obtained in A and emulsified.
C: Component 1 was added to the emulsion obtained in B. The mixture was mixed to give an O/W cream.

[Comparative Example 13]

A: Components 5 to 12 were mixed.
B: Components 2 to 4 were mixed using a three-roll mill.
C: The composition obtained in B was added to the composition obtained in A and emulsified to give an O/W cream.

[Evaluation of feeling on use, and evaluation of water resistance]

The cosmetic compositions were evaluated for the feeling on use (or non-stickiness) and water resistance on

application in the same manner as the evaluation of the aqueous dispersions.

[Evaluation of transparency, and evaluation of feeling on use]

The sample was evaluated for transparency and the feeling on use (or non-stickiness) on application by a panel of 10 professional members according to the evaluation criteria below. The results of the 10 members were averaged, and the transparency and the feeling on use were rated according to the judgment criteria below.

[Evaluation criteria]

5 points: excellent
4 points: good
3 points: moderate
2 points: rather poor
1 point: poor

[Judgment criteria]

A: The average point is 4.5 points or more.
B: The average point is 3.5 points or more and less than 4.5 points.
C: The average point is 2.5 points or more and less than 3.5 points.
D: The average point is less than 2.5 points.

The sample was judged "Pass" when rated with "C" or above.

[Table 5]

| Formulation (%) | | Example 15 | Comparative Example 13 |
|---|---|---|---|
| 1 | Dispersion of Example 1 (50%: amount of component (a)) | 20 | |
| 2 | STR-100W-LP (Note 1) | | 10 |
| 3 | Polyglyceryl-3 disiloxane dimethicone (HLB 10) | | 1.6 |
| 4 | Dimethicone | 5 | 5 |
| 5 | (Ammonium acryloyldimethyltaurate/VP) copolymer | 0.2 | 0.2 |
| 6 | (Sodium acrylate/sodium acryloyldimethyltaurate) copolymer | 2 | 2 |
| 7 | Polyglyceryl-10 myristate | 0.2 | 0.2 |
| 8 | Polyglyceryl-10 diisostearate | 0.8 | 0.8 |
| 9 | BG | 6 | 8 |
| 10 | Alcohol | 6 | 6 |
| 11 | Phenoxyethanol | 0.2 | 0.2 |
| 12 | Water | 59.6 | 66 |
| Total | | 100 | 100 |
| Transparency | | A | D |
| Feeling on use | | A | D |
| Water resistance | | B | D |
| (Note 1) Fine particle of titanium dioxide (labeling name, INCI: Titanium Dioxide) coated with hydrous silica (labeling name, INCI: Hydrated Silica) and further treated with hydrogen dimethicone (labeling name, INCI: Hydrogen Dimethicone) | | | |

Number average primary particle diameter determined by image analysis of a transmission electron photomicrograph: 15 nm (average diameter of 200 particles measured by image analysis of a transmission electron photomicrograph)

As clear from Table 5, the cosmetic composition of Example 15 that involved the dispersion of the present invention had high transparency on application, gave a nonsticky comfortable feeling on use, and was water resistant. In contrast, Comparative Example 13 in which the essential components (a) to (d) of the present invention were added but were not formulated as the aqueous dispersion of the present invention was unsatisfactory in all evaluations.

[Example 16 and Comparative Example 14]

W/O creams were prepared according to the formulations described in Table 6 below.

[Production method]

[Example 16]

A: Components 4 to 8 were mixed.
B: Components 9 to 13 were mixed.
C: Component 1 was added to the composition obtained in B and mixed.
D: The composition obtained in C was added to the composition obtained in A and emulsified to give a W/O cream.

[Comparative Example 14]

A: Components 5 to 8 were mixed.
B: Components 9 to 13 were mixed.
C: Components 2 to 4 were mixed using a three-roll mill. The resultant mixture was mixed with the composition obtained in A.
D: The composition obtained in B was added to the composition obtained in C and emulsified to give a W/O cream.

**[Table** 6]

| Formulation (%) | | Example 16 | Comparative Example 14 |
|---|---|---|---|
| 1 | Dispersion of Example 1 (50%) | 10 | |
| 2 | STR-100W-LP (Note 1) | | 5 |
| 3 | Polyglyceryl-3 disiloxane dimethicone (HLB 10) | | 0.8 |
| 4 | Dimethicone 6 cs | 5 | 5 |
| 5 | Cyclopentasiloxane | 5 | 5 |
| 6 | KSG-710 (Note 2) | 4 | 4 |
| 7 | KSG-19 (Note 3) | 2 | 2 |
| 8 | Polyglyceryl-3 polydimethylsiloxyethyl dimethicone (HLB 1) | 2 | 2 |
| 9 | Glycerin | 8 | 8 |
| 10 | BG | 4 | 5 |
| 11 | Sodium citrate | 0.2 | 0.2 |
| 12 | Sodium chloride | 1 | 1 |
| 13 | Water | 58.8 | 62 |

(continued)

| Formulation (%) | Example 16 | Comparative Example 14 |
|---|---|---|
| Total | 100 | 100 |
| (Note 1) Fine particle of titanium dioxide (labeling name, INCI: Titanium Dioxide) coated with hydrous silica (labeling name, INCI: Hydrated Silica) and further treated with hydrogen dimethicone (labeling name, INCI: Hydrogen Dimethicone)<br>Number average primary particle diameter determined by image analysis of a transmission electron photomicrograph: 15 nm (average diameter of 200 particles measured by image analysis of a transmission electron photomicrograph)<br>(Note 2) Mixture of about 70-80% by weight dimethicone (6 cs) + about 20-30% by weight (dimethicone/polyglycerin-3) crosspolymer, Shin-Etsu Chemical Co., Ltd.<br>(Note 3) Mixture of about 80-90% by weight dimethicone (6 cs) + about 10-20% by weight (dimethicone/vinyl dimethicone) crosspolymer, Shin-Etsu Chemical Co., Ltd. | | |

The cosmetic composition of Example 16 that involved the dispersion of the present invention had high transparency on application, gave a nonsticky comfortable feeling on use, and was water resistant. In contrast, Comparative Example 14 in which the essential components (a) to (d) of the present invention were added but were not formulated as the aqueous dispersion of the present invention was unsatisfactory in all evaluations.

[Example 17] Oil-in-water sunscreen

| | Formulation | % |
|---|---|---|
| 1. | KSG-15 (Note 1) | 8.0 |
| 2. | KSG-16 (Note 2) | 19.0 |
| 3. | KSG-45 (Note 3) | 5.0 |
| 4. | Cyclopentasiloxane | 10.0 |
| 5. | BG | 3.0 |
| 6. | KF-6100 (Note 4) | 0.6 |
| 7. | KF-6104 (Note 5) | 0.3 |
| 8. | Aqueous (ammonium acryloyldimethyltaurate/VP) copolymer solution (Note 6) | 13.0 |
| 9. | Aqueous (acrylamide/sodium acryloyldimethyltaurate)copolymer/ isohexadecane/polysorbate 80 solution (Note 7) | 0.6 |
| 10. | 1% Aqueous sodium chloride solution | 8.0 |
| 11. | Water | 12.5 |
| 12. | Silicone-treated fine particle of titanium dioxide (Note 8) 5%<br>• BG 1%<br>• Polyglyceryl-3 disiloxane dimethicone (HLB 10) 0.7%<br>• Water 3.3% | 10.0 |
| 13. | Silicone-treated fine particle of zinc oxide (Note 9) 5%<br>• BG 1%<br>• Polyglyceryl-3 disiloxane dimethicone (HLB 10) 0.7%<br>• Water 3.3% | 10.0 |

(continued)

| Formulation | % |
|---|---|
| Total | 100.0 |

(Note 1) Mixture of 90-96% by weight cyclopentasiloxane + 4-10% by weight (dimethicone/vinyl dimethicone) crosspolymer, Shin-Etsu Chemical Co., Ltd.
(Note 2) Mixture of 70-80% by weight dimethicone + 20-30% by weight (dimethicone/vinyl dimethicone) crosspolymer, Shin-Etsu Chemical Co., Ltd.
(Note 3) Mixture of 60-70% by weight cocoalkyl (caprylate/caprate) + 30-40% by weight (dimethicone/vinyl dimethicone) crosspolymer, Shin-Etsu Chemical Co., Ltd.
(Note 4) Polyglyceryl-3 disiloxane dimethicone, Shin-Etsu Chemical Co., Ltd.
(Note 5) Polyglyceryl-3 polydimethylsiloxyethyl dimethicone, Shin-Etsu Chemical Co., Ltd.
(Note 6) Aristoflex AVC, Clariant AG
(Note 7) SIMULGEL 600, Seppic
(Note 8) Fine particle of titanium dioxide (labeling name, INCI: Titanium Dioxide) coated with hydrous silica (labeling name, INCI: Hydrated Silica) and further treated with Triethoxysilylethyl Polydimethylsiloxyethylhexyl Dimethicone (INCI)
(Note 9) Treated with AES-3083 (triethoxycaprylylsilane), Shin-Etsu Chemical Co., Ltd.

(Production method)

A: Components 1 to 4 were mixed uniformly.

B: Components 5 to 11 were mixed uniformly.

C: Components 12 and 13 were each uniformly dispersed with a bead mill.

D: The mixture obtained in the step A was added to the mixture obtained in the step B and emulsified, and the dispersions obtained in C were added. The mixture was uniformly dispersed.

The oil-in-water sunscreen obtained as described above showed high stability, high transparency, a nonsticky pleasant feeling on use, and excellent water resistance. Component 12 and Component 13 had excellent stability and were easy to handle and admit.

[Example 18] Oil-in-water base cream

| | Formulation | % |
|---|---|---|
| 1. | Water | Balance |
| 2. | Glycerin | 3.0 |
| 3. | Microcrystalline wax | 3.0 |
| 4. | Xanthan gum | 0.2 |
| 5. | Pentylene glycol | 2.0 |
| 6. | BG | 5.0 |
| 7. | Hydrous silica·hydrogen dimethicone-treated fine particle of titanium dioxide | 5.0 |
| 8. | BG | 1.0 |
| 9. | Polyglyceryl-3 polydimethylsiloxyethyl dimethicone (HLB 12) (Note 1) | 0.1 |
| 10. | Polyglyceryl-3 disiloxane dimethicone (HLB 10) | 0.7 |
| 11. | Water | 3.3 |
| 12. | Sucrose cocoate | 0.2 |
| 13. | Sorbitan stearate | 3.0 |
| 14. | PEG-60 glyceryl isostearate | 0.5 |

(continued)

| | Formulation | % |
|---|---|---|
| 15. | Behenyl alcohol | 0.5 |
| 16. | Ethylhexyl palmitate | 3.0 |
| 17. | KSP-101 (Note 2) | 3.0 |
| 18. | Triethylhexanoin | 6.0 |
| 19. | Polyhydroxystearic acid | 0.5 |
| 20. | KTP-09W (Note 3) | Proper |
| 21. | KTP-09R (Note 3) | Proper |
| 22. | KTP-09Y (Note 3) | Proper |
| 23. | KTP-09B (Note 3) | Proper |
| 24. | Polysorbate 60 | 0.3 |
| 25. | (Hydroxvethvl acrylate/sodium acrvlovldimethvltaurate) copolymer (Note 4) | 0.6 |
| | Total | 100.0 |

(Note 1) Dissolves in water and BG
(Note 2) (Vinyl dimethicone/methicone silsesquioxane) crosspolymer, Shin-Etsu Chemical Co., Ltd.
(Note 3) KF-9909-treated coloring inorganic pigments, W: white, R: red, Y: yellow, B: black, Shin-Etsu Chemical Co., Ltd.
(Note 4) SIMULGEL EG, SEPPIC

(Production method)

A: Components 7 to 11 were dispersed uniformly with a bead mill.

B: Components 1 to 6 were mixed uniformly, and the dispersion obtained in A was added. The resultant mixture was mixed uniformly.

C: Components 12 to 16 were heated and dissolved, and component 17 was added. The resultant mixture was mixed uniformly.

D: Components 18 to 23 were mixed and roll treated.

E: The mixture obtained in the step D was added to the mixture obtained in the step C. The resultant mixture was mixed uniformly.

F: The mixture obtained in the step E was heated and added to the hot mixture obtained in the step B. The resultant mixture was emulsified uniformly.

G: The emulsion obtained in the step F was cooled to room temperature, and subsequently components 24 and 25 were added. The resultant mixture was mixed uniformly.

H: The mixture obtained in the step G was defoamed and then added into a container. An oil-in-water base cream was thus obtained.

The oil-in-water base cream obtained as described above showed high stability, high transparency, a nonsticky pleasant feeling on use, and excellent water resistance. The dispersion obtained in the step A had excellent stability and was easy to handle and admit.

[Example 19] Aqueous gel

| | Formulation | % |
|---|---|---|
| 1. | Dispersion of Example 4 | 5.0 |
| 2. | Ethanol | 3.5 |
| 3. | BG | 4.0 |
| 4. | Glycerin | 2.0 |
| 5. | (Ammonium acryloyldimethyltaurate/VP) copolymer | 0.2 |
| 6. | Xanthan gum | 0.2 |
| 7. | KSP-100W (Note 1) | 1.0 |
| 8. | Phenoxyethanol | 0.3 |
| 9. | Water | Balance |
| | Total | 100.0 |

(Note 1) (Vinyl dimethicone/methicone silsesquioxane) crosspolymer, Shin-Etsu Chemical Co., Ltd.

(Production method)

A: Components 1 and 2 were mixed uniformly.

B: Components 3 to 9 were mixed uniformly.

C: The mixture obtained in the step A was added to the mixture obtained in the step B. The resultant mixture was mixed uniformly.

D: The mixture obtained in the step C was defoamed and then added into a container. An aqueous gel was thus obtained.

The aqueous gel obtained as described above showed full freshness on application, high stability, high transparency, a nonsticky pleasant feeling on use, and excellent water resistance. The component 1 had excellent stability and was easy to handle and admit.

[Example 20] Oil-in-water sunscreen milky lotion

| | Formulation | % |
|---|---|---|
| 1. | Hydrous silica·hydrogen dimethicone-treated fine particle of titanium dioxide | 7.5 |
| 2. | Ethanol | 0.5 |
| 3. | BG | 1.0 |
| 4. | KS-66 (Note 1) | 0.001 |
| 5. | Water | 4.999 |
| 6. | Polyglyceryl-3 disiloxane dimethicone (HLB 10) | 1.2 |
| 7. | Ethylhexylglycerin | 0.1 |
| 8. | Ethanol | 9.5 |
| 9. | BG | 5.0 |
| 10. | Sodium acrylate/sodium acryloyldimethyltaurate copolymer composition (Note 2) | 2.5 |
| 11. | Water | Balance |
| 12. | KF-7312J (Note 3) | 1.0 |
| 13. | KF-56A (Note 4) | 3.0 |
| 14. | KSG-016F (Note 5) | 1.0 |
| 15. | Cetanol | 2.0 |
| 16. | Ethylhexyl methoxycinnamate | 5.0 |
| 17. | Diethylamino hydroxybenzoyl hexyl benzoate | 1.0 |

(continued)

| | Formulation | % |
|---|---|---|
| 18. | Polyoxyethylene (60) hydrogenated castor oil | 1.0 |
| 19. | KF-6011 (Note 6) | 0.5 |
| 20. | Tocopherol | 0.05 |
| | Total | 100.0 |

(Note 1) Simethicone, Shin-Etsu Chemical Co., Ltd.
(Note 2) SIMULGEL EG, SEPPIC
(Note 3) 50% by weight solution of trimethylsiloxysilicic acid in cyclopentasiloxane, Shin-Etsu Chemical Co., Ltd.
(Note 4) Diphenylsiloxyphenyl trimethicone, Shin-Etsu Chemical Co., Ltd.
(Note 5) Mixture of 70-80% by weight dimethicone + 20-30% by weight (dimethicone/vinyl dimethicone) crosspolymer, Shin-Etsu Chemical Co., Ltd.
(Note 6) PEG-11 methyl ether dimethicone, Shin-Etsu Chemical Co., Ltd.

(Production method)

A: Components 1 to 7 were dispersed uniformly using a bead mill.

B: Components 8 to 11 were heated to 85°C, and the dispersion obtained in A was added. The resultant mixture was mixed uniformly.

C: Components 12 to 20 were heated to 85°C and mixed uniformly.

D: The mixture obtained in the step C was added to the mixture obtained in the step B. The resultant mixture was emulsified at 85°C, and then cooled slowly while performing stirring. An oil-in-water sunscreen milky lotion was thus obtained.

The oil-in-water sunscreen milky lotion obtained as described above showed high stability, high transparency, a nonsticky pleasant feeling on use, and excellent water resistance. The dispersion obtained in the step A had excellent stability and was easy to handle and admit.

[Example 21] Oil-in-water primer

| | Formulation | % |
|---|---|---|
| 1. | KP-545 (Note 1) | 3 |
| 2. | KSG-19 (Note 2) | 5 |
| 3. | KF-56A (Note 3) | 5 |
| 4. | Cyclopentasiloxane | 10 |
| 5. | Hydrous silica·hydrogen dimethicone-treated fine particle of titanium dioxide | 7.2 |
| 6. | BG | 1.8 |
| 7. | Polyglyceryl-3 disiloxane dimethicone (HLB 10) | 1.6 |
| 8. | Water | 20 |
| 9. | BG | 10 |
| 10. | Betaine | 1 |
| 11. | KF-6043 (Note 4) | 1.5 |
| 12. | Sodium acrylate-sodium acryloyldimethyltaurate copolymer composition (Note 5) | 1 |
| 13. | (Acrylates/alkyl (C10-30) acrylate) crosspolymer (2% aqueous solution) | 20 |
| 14. | Arginine (10% aqueous solution) | Proper |
| 15. | Bisabolol | 0.1 |
| 16. | Ethylhexylglycerin | 0.1 |

(continued)

| | Formulation | % |
|---|---|---|
| 17. | EDTA-2Na (10% aqueous solution) | 0.1 |
| 18. | Water | Balance |
| | Total | 100.0 |

(Note 1) 30% by weight solution of (acrylates/dimethicone) copolymer in cyclopentasiloxane, Shin-Etsu Chemical Co., Ltd.
(Note 2) Mixture of 80-90% by weight dimethicone + 10-20% by weight (dimethicone/vinyl dimethicone) crosspolymer, Shin-Etsu Chemical Co., Ltd.
(Note 3) Diphenylsiloxyphenyl trimethicone, Shin-Etsu Chemical Co., Ltd.
(Note 4) PEG-10 dimethicone, Shin-Etsu Chemical Co., Ltd.
(Note 5) SIMULGEL EG, SEPPIC

(Production method)

A: Components 1 to 4 were mixed uniformly.

B: Components 5 to 8 were dispersed uniformly using a homomixer.

C: Components 9 to 18 and the dispersion obtained in B were mixed uniformly.

D: The mixture obtained in the step A was added to the mixture obtained in the step C. The resultant mixture was emulsified to give an O/W primer.

The oil-in-water primer obtained as described above showed high stability, high transparency, a nonsticky pleasant feeling on use, and excellent water resistance. The dispersion obtained in the step B had excellent stability and was easy to handle and admit.

[Example 22] Oil-in-water liquid foundation

| | Formulation | % |
|---|---|---|
| 1. | Stearic acid | 1.0 |
| 2. | Behenyl alcohol | 0.4 |
| 3. | Glyceryl stearate | 0.3 |
| 4. | Mineral oil | 10.0 |
| 5. | Glyceryl trioctanoate | 5.0 |
| 6. | KP-561P (Note 1) | 3.0 |
| 7. | Sorbitan sesquioleate | 0.5 |
| 8. | Sorbitan monooleate | 1.0 |
| 9. | Acrylates copolymer | 2.2 |
| 10. | Triethanolamine | 1.0 |
| 11. | KF-6013 (Note 2) | 0.2 |
| 12. | Alkyl-POE palmityl ether phosphate | 0.1 |
| 13. | POE hydrogenated castor oil | 0.5 |
| 14. | Silicone-treated titanium dioxide (Note 3) | 8.5 |
| 15. | Silicone-treated red iron oxide (Note 3) | 0.4 |
| 16. | Silicone-treated yellow iron oxide (Note 3) | 1.0 |
| 17. | Silicone-treated black iron oxide (Note 3) | 0.1 |
| 18. | Hydrous silica·hydrogen dimethicone-treated fine particle of titanium dioxide | 5.0 |

(continued)

| | Formulation | % |
|---|---|---|
| 19. | DPG | 3.0 |
| 20. | Polyglyceryl-3 disiloxane dimethicone (HLB 10) | 0.7 |
| 21. | Water | 3.3 |
| 22. | BG | 7.0 |
| 23. | Preservative | Proper |
| 24. | Fragrance | Proper |
| 25. | Water | Balance |
| | Total | 100.0 |

(Note 1) (Acrylates/stearyl acrylate/dimethicone methacrylate) copolymer, Shin-Etsu Chemical Co., Ltd.
(Note 2) PEG-9 dimethicone, Shin-Etsu Chemical Co., Ltd.
(Note 3) Treated with triethoxycaprylylsilane, Shin-Etsu Chemical Co., Ltd.

(Production method)

A: Components 11 to 13 were mixed with a portion of component 22, and components 14 to 17 were added. The resultant mixture was dispersed uniformly and heated.

B: Components 1 to 8 were mixed and heated to give a uniform solution.

C: Components 9 and 10, the remainder of component 22, and components 23 and 25 were mixed and heated.

D: Components 18 to 21 were dispersed using a disperser.

E: While performing stirring, the mixture obtained in the step B was added to the mixture obtained in the step C and emulsified. The dispersion obtained in the step A was added, and further the dispersion obtained in the step D and component 24 were added. An oil-in-water liquid foundation was thus obtained.

The oil-in-water liquid foundation obtained as described above showed high stability, high transparency, a nonsticky pleasant feeling on use, and excellent water resistance. The dispersion obtained in the step D had excellent stability and was easy to handle and admit.

[Example 23] Water-in-oil sunscreen milky lotion

| | Formulation | % |
|---|---|---|
| 1. | KSG-210 (Note 1) | 2.0 |
| 2. | KSG-790 (Note 2) | 1.0 |
| 3. | KSG-15 (Note 3) | 2.0 |
| 4. | KF-6028 (Note 4) | 1.0 |
| 5. | Dimethicone 6 CS | 3.0 |
| 6. | KF-4418 (Note 5) | 2.0 |
| 7. | Cyclopentasiloxane | 5.0 |
| 8. | Isotridecyl isononanoate | 4.0 |
| 9. | SPD-T5 (Note 6) | 5.0 |
| 10. | SPD-Z5 (Note 7) | 15.0 |
| 11. | SPD-Z7L (Note 8) | 20.0 |
| 12. | Sodium citrate | 0.2 |
| 13. | Sodium chloride | 0.5 |

(continued)

| | Formulation | % |
|---|---|---|
| 14. | Dispersion of Example 5 | 20.0 |
| 15. | Preservative | Proper |
| 16. | Fragrance | Proper |
| 17. | Water | Balance |
| | Total | 100.0 |

(Note 1) Mixture of 70-80% by weight dimethicone + 20-30% by weight (dimethicone/(PEG-10/15)) crosspolymer, Shin-Etsu Chemical Co., Ltd.
(Note 2) Mixture of 65-75% by weight caprylyl methicone + 25-35% by weight (dimethicone/polyglycerin-3) crosspolymer, Shin-Etsu Chemical Co., Ltd.
(Note 3) Mixture of 90-96% by weight cyclopentasiloxane + 4-10% by weight (dimethicone/vinyl dimethicone) crosspolymer, Shin-Etsu Chemical Co., Ltd.
(Note 4) PEG-9 polydimethylsiloxyethyl dimethicone, Shin-Etsu Chemical Co., Ltd.
(Note 5) Caprylyl methicone, Shin-Etsu Chemical Co., Ltd.
(Note 6) 40% by weight dispersion of fine particle of titanium dioxide in cyclopentasiloxane, Shin-Etsu Chemical Co., Ltd.
(Note 7) 60% by weight dispersion of fine particle of zinc oxide in cyclopentasiloxane, Shin-Etsu Chemical Co., Ltd.
(Note 8) 60% by weight dispersion of fine particle of zinc oxide in dimethicone, Shin-Etsu Chemical Co., Ltd.

(Production method)

A: Components 1 to 8 were mixed uniformly.

B: Components 12 to 17 were mixed uniformly.

C: While performing stirring, the mixture obtained in the step A was added to the mixture obtained in the step B and emulsified. Components 9 to 11 were then added. A water-in-oil sunscreen milky lotion was thus obtained.

The water-in-oil sunscreen milky lotion obtained as described above showed high stability, high transparency, a nonsticky pleasant feeling on use, and excellent water resistance. Component 14 had excellent stability and was easy to handle and admit.

[Example 24] Oil-in-water sunscreen milky lotion

| | Formulation | % |
|---|---|---|
| 1. | Hydrous silica·hydrogen dimethicone-treated fine particle of titanium dioxide | 10 |
| 2. | BG | 2 |
| 3. | Polyglyceryl-3 disiloxane dimethicone (HLB 10) | 1.4 |
| 4. | KM-72 (Note 1) | 0.001 |
| 5. | Water | 6.599 |
| 6. | Cyclopentasiloxane | 10 |
| 7. | KF-56A (Note 2) | 3 |
| 8. | Cetanol | 0.5 |
| 9. | Polyoxyethylene sorbitan monooleate | 2.5 |
| 10. | Glyceryl stearate (SE) | 0.5 |
| 11. | KF-6011 (Note 3) | 1 |
| 12. | DPG | 6 |
| 13. | BG | 6 |
| 14. | Carboxyvinyl polymer | 0.3 |

(continued)

| | Formulation | % |
|---|---|---|
| 15. | Acrylic polymer compound | 0.3 |
| 16. | Methylparaben | 0.2 |
| 17. | Phenoxyethanol | 0.3 |
| 18. | EDTA-2Na | Proper |
| 19. | Water | Balance |
| 20. | 10% Aqueous sodium hydroxide solution | Proper |
| 21. | KM-116 (Note 4) | 2 |
| | Total | 100.0 |

(Note 1) Simethicone emulsion, Shin-Etsu Chemical Co., Ltd.
(Note 2) Diphenylsiloxyphenyl trimethicone, Shin-Etsu Chemical Co., Ltd.
(Note 3) PEG-11 methyl ether dimethicone, Shin-Etsu Chemical Co., Ltd.
(Note 4) (Dimethicone/vinyl dimethicone) crosspolymer emulsion, Shin-Etsu Chemical Co., Ltd.

(Production method)

A: Components 1 to 5 were dispersed uniformly using a high-pressure disperser.

B: Components 6 to 8 were mixed uniformly.

C: Components 9 to 19 and the dispersion obtained in A were mixed uniformly.

D: The mixture obtained in the step B was added to the mixture obtained in the step C. The mixture was emulsified, and component 20 was added. The resultant mixture was mixed uniformly.

E: Component 21 was added to the mixture obtained in the step D. The resultant mixture was mixed uniformly.

The oil-in-water sunscreen milky lotion obtained as described above showed high stability, high transparency, a nonsticky pleasant feeling on use, and excellent water resistance. The dispersion obtained in the step A had excellent stability and was easy to handle and admit.

[Example 25] Oil-in-water sunscreen milky lotion

| | Formulation | % |
|---|---|---|
| 1. | TSPL-30-D5 (Note 1) | 3 |
| 2. | KSG-18A (Note 2) | 5 |
| 3. | KF-56A (Note 3) | 5 |
| 4. | Cyclopentasiloxane | 10 |
| 5. | Hydrous silica·hydrogen dimethicone-treated fine particle of titanium dioxide | 10 |
| 6. | BG | 2 |
| 7. | Polyglyceryl-3 disiloxane dimethicone (HLB 10) | 1.6 |
| 8. | Water | 20 |
| 9. | Sodium chloride | 0.2 |
| 10. | BG | 10 |
| 11. | KF-6043 (Note 4) | 1.5 |
| 12. | Sodium acrylate-sodium acryloyldimethyltaurate copolymer composition (Note 5) | 1 |
| 13. | (Acrylates/alkyl (C10-30) acrylate) crosspolymer (2% aqueous solution) | 20 |
| 14. | Arginine (10% aqueous solution) | Proper |

(continued)

| | Formulation | % |
|---|---|---|
| 15. | EDTA-2Na (10% aqueous solution) | 0.1 |
| 16. | Water | Balance |
| | Total | 100.0 |

(Note 1) 30% Solution of tri(trimethylsiloxy)silylpropylcarbamoyl pullulan in cyclopentasiloxane, Shin-Etsu Chemical Co., Ltd.
(Note 2) Mixture of 80-90% by weight diphenylsiloxyphenyl trimethicone + 10-20% (dimethicone/phenyl vinyl dimethicone) crosspolymer, Shin-Etsu Chemical Co., Ltd.
(Note 3) Diphenylsiloxyphenyl trimethicone, Shin-Etsu Chemical Co., Ltd.
(Note 4) PEG-10 dimethicone, Shin-Etsu Chemical Co., Ltd.
(Note 5) SEPILIFE NUDE, SEPPIC

(Production method)

A: Components 1 to 4 were mixed uniformly.

B: Components 5 to 9 were dispersed uniformly using a paint shaker.

C: Components 10 to 16 were mixed uniformly.

D: The mixture obtained in the step A was added to the mixture obtained in the step C and emulsified, and the dispersion obtained in the step B was mixed therewith to give a sunscreen milky lotion.

The sunscreen milky lotion obtained as described above showed high stability, high transparency, a nonsticky pleasant feeling on use, and excellent water resistance. The dispersion obtained in the step B had excellent stability and was easy to handle and admit.

[Example 26] Aqueous sunscreen mist

| | Formulation | % |
|---|---|---|
| 1. | Dispersion of Example 4 | 15.0 |
| 2. | Ethanol | 5.0 |
| 3. | BG | 4.0 |
| 4. | Glycerin | 2.0 |
| 5. | Sodium citrate | 0.1 |
| 6. | Citric acid | 0.02 |
| 7. | Fragrance | 0.2 |
| 8. | Ethylhexylglycerin | 0.1 |
| 9. | Water | Balance |
| | Total | 100.0 |

(Production method)

A: Components 2, 3, 4, 7, and 8 are mixed uniformly.

B: Components 1, 5, 6, and 9 are mixed uniformly.

C: The mixture obtained in the step A is added to the mixture obtained in the step B. The resultant mixture is mixed uniformly.

D: The mixture obtained in the step C is added into a container. An aqueous sunscreen mist was thus obtained.

The aqueous sunscreen mist obtained as described above showed full freshness on application, high stability, high transparency, a nonsticky pleasant feeling on use, and excellent water resistance. Component 1 had excellent stability and was easy to handle and admit.

[Example 27] W/O liquid foundation

| | Formulation | % |
|---|---|---|
| 1. | KSG-210 (Note 1) | 3.0 |
| 2. | KSG-15 (Note 2) | 5.0 |
| 3. | KF-6038 (Note 3) | 3.0 |
| 4. | Dimethicone 6 CS | 13.8 |
| 5. | Ethylhexyl methoxycinnamate | 5.0 |
| 6. | Diethylaminohydroxybenzoylbenzoic acid | 2.0 |
| 7. | KF-6115 (Note 4) | 1.5 |
| 8. | Silicone-treated titanium dioxide (Note 5) | 8.5 |
| 9. | Silicone-treated red iron oxide (Note 5) | 0.4 |
| 10. | Silicone-treated yellow iron oxide (Note 5) | 1.0 |
| 11. | Silicone-treated black iron oxide (Note 5) | 0.1 |
| 12. | Sodium citrate | 0.2 |
| 13. | Sodium chloride | 0.5 |
| 14. | Hydrous silica/aluminum hydroxide/hydrogen dimethicone-treated fine particle of titanium dioxide | 10 |
| 15. | BG | 2 |
| 16. | Polyglyceryl-3 disiloxane dimethicone (HLB 10) | 1.6 |
| 17. | Water | 20 |
| 18. | Methylparaben | Proper |
| 19. | Fragrance | Proper |
| 20. | BG | 2 |
| 21. | Water | Balance |
| | Total | 100.0 |

(Note 1) Mixture of 70-80% dimethicone + 20-30% (dimethicone/(PEG-10/15)) crosspolymer, Shin-Etsu Chemical Co., Ltd.
(Note 2) Mixture of 90-96% cyclopentasiloxane + 4-10% (dimethicone/vinyl dimethicone) crosspolymer, Shin-Etsu Chemical Co., Ltd.
(Note 3) Lauryl PEG-9 polydimethylsiloxyethyl dimethicone, Shin-Etsu Chemical Co., Ltd.
(Note 4) Lauryl polyglyceryl-3 polydimethylsiloxyethyl dimethicone, Shin-Etsu Chemical Co., Ltd.
(Note 5) Treated with hydrogen dimethicone, Shin-Etsu Chemical Co., Ltd.

(Production method)

A: A portion of component 4, and components 7 to 11 were mixed uniformly with a roll mill.

B: Components 14 to 17 were dispersed uniformly using a paint shaker.

C: Components 1 to 6 were mixed uniformly, and the mixture obtained in A was added. The resultant mixture was

mixed uniformly.

D: Components 18 to 20 were mixed uniformly, and components 21, 12, and 13, and the dispersion obtained in B were added. The resultant mixture was mixed uniformly.

E: The mixture obtained in the step D was added to the mixture obtained in the step C and emulsified to give a W/O liquid foundation.

The W/O liquid foundation obtained as described above showed high stability, high transparency, a nonsticky pleasant feeling on use, and excellent water resistance. The dispersion obtained in the step B had excellent stability and was easy to handle and admit.

[Example 28] Oil-in-water sunscreen cream

| | Formulation | % |
|---|---|---|
| 1. | Hydrous silica·hydrogen dimethicone-treated fine particle of titanium dioxide | 7.5 |
| 2. | Pentylene glycol | 0.5 |
| 3. | BG | 1.0 |
| 4. | KS-66 (Note 1) | 0.001 |
| 5. | Water | 4.999 |
| 6. | (c) Silicone of Production Example 4 (HLB 10) | 1.0 |
| 7. | (c) Isostearyl polyglyceryl-3 dimethicone (HLB 14.5) | 0.3 |
| 8. | Ethanol | 9.5 |
| 9. | BG | 5.0 |
| 10. | (Hydroxyethyl acrylate/sodium acryloyldimethyltaurate) copolymer mixture liquid (Note 2) | 2.0 |
| 11. | Aqueous xanthan gum solution | 2.5 |
| 12. | Water | Balance |
| 13. | KF-7312T (Note 3) | 1.0 |
| 14. | TMF-1.5 (Note 4) | 3.0 |
| 15. | Ethylhexyl methoxycinnamate | 5.0 |
| 16. | Ethylhexyl triazone | 0.5 |
| 17. | Polyglyceryl-10 myristate | 0.2 |
| 18. | Polyglyceryl-10 diisostearate | 0.8 |
| 19. | KF-6043 (Note 5) | 0.5 |
| 20. | Phenoxyethanol | 0.3 |
| | Total | 100.0 |

(Note 1) Simethicone, Shin-Etsu Chemical Co., Ltd.
(Note 2) SIMULGEL NS, SEPPIC
(Note 3) 50% by weight solution of trimethylsiloxysilicic acid in methyl trimethicone, Shin-Etsu Chemical Co., Ltd.
(Note 4) Methyl trimethicone, Shin-Etsu Chemical Co., Ltd.
(Note 5) PEG-10 dimethicone, Shin-Etsu Chemical Co., Ltd.

(Production method)

A: Components 1 to 7 were dispersed uniformly using a bead mill.

B: Components 8 to 12 were heated to 85°C, and the dispersion obtained in A was added. The resultant mixture was mixed uniformly.

C: Components 13 to 20 were heated to 85°C and mixed uniformly.

D: The mixture obtained in the step C was added to the mixture obtained in the step B and emulsified at 85°C. While performing stirring, the emulsion was cooled gradually. An oil-in-water sunscreen cream was thus obtained.

The oil-in-water sunscreen cream obtained as described above showed high stability, high transparency, a nonsticky pleasant feeling on use, and excellent water resistance. The dispersion obtained in the step A had excellent stability and was easy to handle and admit.

[Example 29] Water-in-oil sunscreen cream

| | Formulation | % |
|---|---|---|
| 1. | KSG-270 (Note 1) | 2.0 |
| 2. | KSG-790 (Note 2) | 1.0 |
| 3. | KSG-45 (Note 3) | 2.0 |
| 4. | KF-6048 (Note 4) | 1.0 |
| 5. | Cocoalkyl (caprylate/caprate) | 3.0 |
| 6. | KF-4418 (Note 5) | 5.0 |
| 7. | Squalane | 2.0 |
| 8. | KMP-592 (Note 6) | 1.0 |
| 9. | SPD-T5L (Note 7) | 5.0 |
| 10. | SPD-Z7L (Note 8) | 20.0 |
| 11. | Hydrous silica·hydrogen dimethicone-treated fine particle of titanium dioxide | 7.5 |
| 12. | Glycerin | 1.3 |
| 13. | Pentylene glycol | 0.2 |
| 14. | KS-69 (Note 9) | 0.001 |
| 15. | Silicone of Production Example 3 (HLB 10) | 1.0 |
| 16. | Sodium citrate | 0.2 |
| 17. | Sodium chloride | 0.5 |
| 18. | Water | 4.999 |
| 19. | BG | 5.0 |
| 20. | Preservative | Proper |
| 21. | Fragrance | Proper |
| 22. | Water | Balance |
| | Total | 100.0 |

(Note 1) Mixture of 75-85% diphenylsiloxyphenyl trimethicone + 15-25% (dimethicone/(PEG-10/15)) crosspolymer, Shin-Etsu Chemical Co., Ltd.
(Note 2) Mixture of 65-75% caprylyl methicone + 25-35% (dimethicone/polyglycerin-3) crosspolymer, Shin-Etsu Chemical Co., Ltd.
(Note 3) Mixture of 60-70% cocoalkyl (caprylate/caprate) + 30-40% (dimethicone/vinyl dimethicone) crosspolymer, Shin-Etsu Chemical Co., Ltd.
(Note 4) Cetyl PEG/PPG-10/1 dimethicone, Shin-Etsu Chemical Co., Ltd.
(Note 5) Caprylyl methicone, Shin-Etsu Chemical Co., Ltd.
(Note 6) (Methyl/phenyl) polysilsesquioxane, Shin-Etsu Chemical Co., Ltd.
(Note 7) 40% Dispersion of fine particle of titanium dioxide in dimethicone, Shin-Etsu Chemical Co., Ltd.
(Note 8) 60% Dispersion of fine particle of zinc oxide in dimethicone, Shin-Etsu Chemical Co., Ltd.
(Note 9) Simethicone, Shin-Etsu Chemical Co., Ltd.

(Production method)

A: Components 11 to 15 were dispersed uniformly using a bead mill.

B: Components 1 to 7 were mixed uniformly.

C: Components 16 to 22 were mixed uniformly.

D: While performing stirring, the mixture obtained in the step C was added to the mixture obtained in the step B and emulsified. The dispersion obtained in A and components 8 to 10 were added. A water-in-oil sunscreen milky lotion was thus obtained.

The water-in-oil sunscreen milky lotion obtained as described above showed high stability, high transparency, a nonsticky pleasant feeling on use, and excellent water resistance. The dispersion obtained in the step A had excellent stability and was easy to handle and admit.

[Example 30] Aqueous sunscreen gel

| | Formulation | % |
|---|---|---|
| 1. | Hydrous silica·hydrogen dimethicone-treated fine particle of titanium dioxide | 7.5 |
| 2. | Diglycerin | 0.2 |
| 3. | BG | 1.3 |
| 4. | KS-66 (Note 1) | 0.001 |
| 5. | Water | 4.999 |
| 6. | Silicone of Production Example 1 (HLB 8) | 0.5 |
| 7. | Silicone of Production Example 2 (HLB 12) | 0.5 |
| 8. | BG | 4.0 |
| 9. | Glycerin | 2.0 |
| 10. | (PEG-240/decyltetradeceth-20/HDI) copolymer | 0.2 |
| 11. | Xanthan gum | 0.2 |
| 12. | KSP-100W (Note 2) | 1.0 |
| 13. | Phenoxyethanol | 0.3 |
| 14. | Water | Balance |
| | Total | 100.0 |

(Note 1) Simethicone, Shin-Etsu Chemical Co., Ltd.
(Note 2) (Vinyl dimethicone/methicone silsesquioxane) crosspolymer, Shin-Etsu Chemical Co., Ltd.

(Production method)

A: Components 1 to 9 were dispersed uniformly with a bead mill.

B: Components 10 to 14 were mixed uniformly.

C: The dispersion obtained in the step A was added to the mixture obtained in the step B. The resultant mixture was mixed uniformly.

D: The mixture obtained in the step C was defoamed and then added into a container. An aqueous sunscreen gel was thus obtained.

The aqueous sunscreen gel obtained as described above showed full freshness on application, high stability, high transparency, a nonsticky pleasant feeling on use, and excellent water resistance. The dispersion obtained in the step A had excellent stability and was easy to handle and admit.

## Claims

1. An aqueous dispersion comprising:

   (a) 10 to 70% by weight of hydrophobized titanium dioxide fine particles including titanium dioxide particles hydrophobized with a silicone, the hydrophobized titanium dioxide fine particles having a number average primary particle diameter of 8 to 200 nm as determined by image analysis of a transmission electron photomicrograph;
   (b) 1.0 to 30% by weight of an aqueous component having at least two alcoholic hydroxyl groups;
   (c) 1.0 to 20% by weight of a polyglycerin-modified silicone that dissolves in the component (b); and
   (d) 8 to 82% by weight of water.

2. The aqueous dispersion according to claim 1, wherein the component (a) is a hydrophobized fine particle of titanium dioxide obtained by hydrophobizing hydrous silica-coated titanium dioxide particles.

3. The aqueous dispersion according to claim 1, wherein the silicone in the component (a) is hydrogen dimethicone.

4. The aqueous dispersion according to claim 1, wherein the component (b) is an aqueous component having two alcoholic hydroxyl groups.

5. The aqueous dispersion according to claim 1, wherein the component (c) is a component that does not dissolve in the component (d).

6. The aqueous dispersion according to claim 1, wherein the component (c) is polyglyceryl-3 disiloxane dimethicone.

7. The aqueous dispersion according to claim 1, wherein the weight ratio (c)/(b) of the content of the component (c) to the content of the component (b) is 0.2 to 0.9.

8. The aqueous dispersion according to claim 1, wherein the total content of the components (a), (b), (c), and (d) is 90% by weight or more of the whole of the aqueous dispersion.

9. An aqueous dispersion comprising:

   (a) 10 to 70% by weight of hydrophobized titanium dioxide fine particles having a number average primary particle diameter of 8 to 200 nm as determined by image analysis of a transmission electron photomicrograph, the hydrophobized titanium dioxide fine particles being such that titanium dioxide particles selected from hydrous silica-coated titanium dioxide particles, and hydrous silica-coated and aluminum hydroxide-coated titanium dioxide particles are hydrophobized with a silicone selected from triethoxycaprylylsilane, dimethyl silicone, hydrogen dimethicone, triethoxysilylethyl polydimethylsiloxyethyl dimethicone, and triethoxysilylethyl polydimethylsiloxyethylhexyl dimethicone;
   (b) 1.0 to 30% by weight of at least one selected from BG (labeling name), DPG (labeling name), and glycerin;
   (c) 1.0 to 20% by weight of polyglyceryl-3 disiloxane dimethicone; and
   (d) 8 to 82% by weight of water.

10. A cosmetic composition comprising the aqueous dispersion described in any one of claims 1 to 9.

## INTERNATIONAL SEARCH REPORT

International application No. **PCT/JP2024/034121**

**A. CLASSIFICATION OF SUBJECT MATTER**

***A61K 8/894***(2006.01)i; ***A61K 8/04***(2006.01)i; ***A61K 8/19***(2006.01)i; ***A61K 8/29***(2006.01)i; ***A61K 8/34***(2006.01)i; ***A61K 8/891***(2006.01)i; ***A61Q 17/04***(2006.01)i; ***A61Q 19/00***(2006.01)i
FI: A61K8/894; A61K8/04; A61K8/29; A61K8/891; A61Q19/00; A61K8/34; A61K8/19; A61Q17/04

According to International Patent Classification (IPC) or to both national classification and IPC

**B. FIELDS SEARCHED**

Minimum documentation searched (classification system followed by classification symbols)

A61K8/894; A61K8/04; A61K8/19; A61K8/29; A61K8/34; A61K8/891; A61Q17/04; A61Q19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2023/132256 A1 (SHIN-ETSU CHEMICAL CO., LTD.) 13 July 2023 (2023-07-13) claims, paragraph [0007], examples 1-6 | 1-5, 7-8, 10<br>2, 9-10 |
| X<br>Y | JP 2014-201569 A (SHIN-ETSU CHEMICAL CO., LTD.) 27 October 2014 (2014-10-27) claims, paragraphs [0023]-[0024], examples 1-4, 6-7, tables 1, 5-6 | 1, 3-8, 10<br>2, 9-10 |
| A | JP 2008-56535 A (TITAN KOGYO KABUSHIKI KAISHA) 13 March 2008 (2008-03-13) entire text, all drawings | 1-10 |
| A | JP 2008-179628 A (KOSE CORP.) 07 August 2008 (2008-08-07) entire text, all drawings | 1-10 |
| A | WO 2023/118916 A1 (L V M H RECHERCHE) 29 June 2023 (2023-06-29) entire text, all drawings | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 October 2024** | **05 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/034121**

**C. DOCUMENTS CONSIDERED TO BE RELEVANT**

| Category* | Citation of document, with indication, where appropriate, of the relevant passages | Relevant to claim No. |
|---|---|---|
| A | WO 2016/000145 A1 (L'OREAL) 07 January 2016 (2016-01-07) entire text, all drawings | 1-10 |
| A | JP 2009-084232 A (POLA CHEMICAL INDUSTRIES, INC.) 23 April 2009 (2009-04-23) entire text, all drawings | 1-10 |
| A | JP 2006-213619 A (FANCL CORP.) 17 August 2006 (2006-08-17) entire text, all drawings | 1-10 |
| A | JP 2010-159229 A (TOKIWA CORP.) 22 July 2010 (2010-07-22) entire text, all drawings | 1-10 |

Form PCT/ISA/210 (second sheet) (July 2022)

**INTERNATIONAL SEARCH REPORT**
**Information on patent family members**

International application No.
**PCT/JP2024/034121**

| Patent document cited in search report | Publication date (day/month/year) | Patent family member(s) | Publication date (day/month/year) |
|---|---|---|---|
| WO 2023/132256 A1 | 13 July 2023 | (Family: none) | |
| JP 2014-201569 A | 27 October 2014 | CN 104095772 A | |
| JP 2008-56535 A | 13 March 2008 | (Family: none) | |
| JP 2008-179628 A | 07 August 2008 | (Family: none) | |
| WO 2023/118916 A1 | 29 June 2023 | (Family: none) | |
| WO 2016/000145 A1 | 07 January 2016 | CN 106659671 A | |
| JP 2009-084232 A | 23 April 2009 | (Family: none) | |
| JP 2006-213619 A | 17 August 2006 | (Family: none) | |
| JP 2010-159229 A | 22 July 2010 | US 2010/0172850 A1<br>entire text, all drawings<br>CN 101773455 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006001886 A
- JP 2014201569 A
- WO 2016178380 A
- WO 2015125622 A
- JP 2020002031 A